# EUROPEAN PATENT SPECIFICATION

(11) **EP 3 293 511 B1**
(45) Date of publication and mention of the grant of the patent: **28.07.2021**
(21) Application number: 17190066.5
(22) Date of filing: 08.09.2017
(51) Int. Cl.: G01N 21/77, A61M 1/16, G01N 31/22, G01N 21/80, G01N 21/78, G01N 33/00

(54) **FLUID SENSOR CARD**
FLÜSSIGKEITSSENSORKARTE
CARTE DE CAPTEUR DE FLUIDE

(30) Priority: 09.09.2016 US 201662385940 P; 15.08.2017 US 201715677338
(43) Date of publication of application: 14.03.2018
(60) Divisional application: 21180141.0
(73) Proprietor: Medtronic, Inc., Minneapolis, Minnesota 55432 (US)
(72) Inventor: LURA, David B., Maple Grove, MN Minnesota 55311 (US); KELLEY, Shawn, Shoreview, MN Minnesota 55126 (US)
(74) Representative: Maschio & Soames IP Ltd

(56) References cited:
- WO-A1-98/54563
- WO-A1-2014/121161
- US-A- 3 754 867
- US-A- 4 772 560
- US-A1- 2005 214 863
- US-A1- 2005 265 895
- US-A1- 2007 031 972
- US-A1- 2007 161 113
- US-A1- 2007 222 992
- US-A1- 2011 081 728
- US-A1- 2014 314 625

## Description

### CROSS-REFERENCE TO RELATED APPLICATIONS

This application claims the benefit of and priority to U.S. Provisional Patent Application No. 62/385,940 filed September 9, 2016.

### FIELD OF THE INVENTION

The invention relates to sensor cards for determining and/or monitoring solute concentration and/or pH of a fluid based on an optically observable change of a sensing membrane or colorimetric material in the presence of ions in solution. The sensor cards can be placed in a fluid sensor apparatus and used to determine the solute concentration and/or pH of any fluid, such as dialysate.

### BACKGROUND

A total ammonia content of a fluid can be determined by either ammonia or ammonium ion concentration along with pH. Known methods and devices for detecting the ph and/or ammonia concentration commonly include materials having a variable output parameter depending on the measured solute in the sampled fluid. An ammonia sensor used by known systems has a chemical substance that changes color or color intensity if exposed to ammonia. Typically, the systems and methods detect the measured solute by submersing the sensor in a static pool of fluid, for example, when measuring ammonia levels in an aquarium. For applications requiring continuous or intermittent measurement of flowing fluid, the systems rely on housings to position a sensor in fluid contact with a flow path. The systems then direct a light source onto the sensor and measure the light reflected off the sensor using an optical detector.

However, the systems and methods do not provide even distribution of the sampled fluid across an entire surface of both sides of the sensor. Also, the systems and methods are restricted to measurements reflected from the same surface on which the emitted light is cast and cannot detect light transmitted through the sensor material. The systems typically access the sensor via an access port that requires a seal between the sensor and a housing to prevent fluid from flowing out of the access port. The access port limits the exposed sensor surface to a single side of the sensor because one side of the sensor surface must be positioned parallel to the direction of flow while an opposing side of the sensor surface must be made accessible to the access port, limiting optically observable detection to one side of a sensor material and restricts the detectable observation to light reflected off the same surface exposed to the fluid flow.

US 3,754,867 A describes a device for detecting components such as carbon dioxide. It comprises a transparent layer containing a reagent which reacts with the component. The extent of the reaction between the component and reagent is measured either by the transmitted or reflected light recorded by a photoelectric system. US 2005/0265895 A1 is directed at a device for measuring the pH of aquatic environments, comprising a sensor card with a colorimetric sensing membrane and a color reference chart. WO 98/54563 A1 is directed at a diagnostic apparatus for analysing saliva. It describes two sample wells which are kept between two supporting layers with holes. US 2007/0031972 A1 relates to systems and methods for the detection poisons in liquid food and/or water samples. The systems preferably include an inexpensive and disposable laminated card including a dry chemical system of detection. The card may have a sample injection port, a reference port, and a QA port. An electronic reader may be used to compare the color of the sample injection port and the reference port to quantitate the concentration of poison in the sample. US 2007/0222992 A1 discloses a colour identifying device comprising a reactive board which comprises a plurality of reaction surfaces. US 4,772,560 A describes a colorimetric gas monitoring wafer for measuring gas concentrations and/or cumulative exposure dose of a selected gas. US 2011/0081728 A1 describes a device for proximate, simultaneous and continuous measuring of a combination of solution pH and ammonia in an aqueous environment e.g. aquarium, comprising a submersible member. WO 2014/121161 A1 is directed at systems and methods for utilizing a sorbent cartridge and quantifying chemistry changes that occur as dialysate passes through material layers of the cartridge as a means to determine solute concentrations in the solution flowing through the cartridge. US 2007/0161113 A1 relates to the use of chemical based sensors to detect ammonia gas in fluids.

Hence, there is a need for a sensor card containing sensor membranes capable of being placed in a fluid flow path and accurately detecting both the ph and/or ammonia concentration of the fluid. The need extends to a sensor card that contacts a flowing fluid on both sides of the sensor card. The need includes receiving light on one side of the sensor membrane and detecting the visual output on the other side of the sensor membrane. There is further a need for a system that provides for a disposable sensor card within a reusable apparatus, decreasing the chances of contamination when used with multiple fluid flow paths. There is further a need for a ph and/or ammonia sensor capable of returning consistent results across multiple replaceable sensor cards and different lots of sensor films, which can vary from lot to lot.

### SUMMARY OF THE INVENTION

According to a first aspect of the invention, there is provided a system for determining solute concentration and/or pH of a fluid according to claim 1.

In any embodiment, the sensor card can include a back carrier overlaying a back side of the at least one fluid sensor membrane; at least a second sampling hole positioned on the back carrier aligned over the back side of the fluid sensor membrane; the first and second sampling holes opposedly positioned on the sensor card.

In any embodiment, the at least one fluid sensor membrane can be selected from the group of a pH sensor membrane, a low sensitivity ammonia sensor membrane, and a high sensitivity ammonia sensor membrane.

In any embodiment, a first sampling hole can face a light emitting source, and a second sampling hole can face a camera or photodetector.

In any embodiment, at least one sampling hole can be positioned on at least one perimeter of a circle having a radius about an axis perpendicular to the sensor card.

In any embodiment, the axis perpendicular to the sensor card can be substantially aligned to a perpendicular center axis of a lens of a photo-detector or a camera.

In any embodiment, at least two sampling holes can be positioned concentrically about an axis perpendicular to the sensor card at different radii.

In any embodiment, at least two sampling holes can be positioned symmetrically about an axis perpendicular to the sensor card.

In any embodiment, the sampling holes can have a shape selected from the group of rectangular, ovoid, circular, triangular, arced, and combinations thereof.

In any embodiment, the sensor card can be substantially rectangular.

In any embodiment, the sensor card can have at least one tapered edge.

In any embodiment, the pH sensor membrane can detect pH in a range of 6.8 to 7.8 the high sensitivity ammonia sensor membrane can detect ammonia in a range of 1 ppm to 2 ppm, and the low sensitivity ammonia sensor membrane can detect ammonia in a range of 1 ppm to 20 ppm.

In any embodiment, the colorimetric material can detect any one of alkalinity, aluminum, ammonium, calcium, carbonate, chloride, chlorine, chlorine dioxide, chromate, color, copper, cyanide, fluoride, formaldehyde, hydrazine, iron, magnesium, manganese, nickel, nitrate, nitrite, oxygen, ozone, pH, phosphate, residual hardness, silicate, sulfate, sulfide, sulfite, total hardness, urea, zinc, or combinations thereof.

In any embodiment, the sensor card can include a first adhesive interposed between the front carrier and the at least one fluid sensor membrane; and a second adhesive layer interposed between the back carrier and the at least one fluid sensor membrane.

In any embodiment, the first adhesive and second adhesive can have a hole cut-out aligned to the first and second sampling holes.

In any embodiment, the sensor card can include a pressure equalizing hole positioned through the sensor card.

In any embodiment, the sensor card can have a thickness of between 0.5 and 3.0 mm.

In any embodiment, the front and back carrier can be polypropylene, polyvinyl chloride, dyed polytetrafluoroethylene, ethylene tetrafluoroethylene, polyvinylidene difluoride, fluorinated ethylene propylene, polyethylene, polyimide, polyetheretherketone, or combinations thereof.

In any embodiment, the sensor card can include a bar code fixed on either a surface of the front carrier or the back carrier.

In any embodiment, the front or back carrier, or both can non-reflective.

Any of the features disclosed as being part of the first aspect of the invention can be included in the first aspect of the invention, either alone or in combination.

According to a second aspect of the invention, there is provided a method according to claim 12.

In any embodiment, the method can include the step of determining any one of a pH or ammonia concentration based on the optically observable change of the sensor membrane.

In any embodiment, the optically observable change can be color or intensity of light.

In any embodiment, the method can include uniformly transmitting the light onto the one side of the sensor membrane. The method of uniformly transmitting the light can use an LED array.

In any embodiment, the fluid can be dialysate and the at least two sensor membranes can be in fluid contact with a dialysate flow path.

In any embodiment, the method can include the step of flowing the dialysate through a sorbent cartridge prior to flowing the dialysate over opposite sides of the at least two sensor membranes.

Any of the features disclosed as being part of the second aspect of the invention can be included in the second aspect of the invention, either alone or in combination.

### BRIEF DESCRIPTION OF THE DRAWINGS

FIG. 1 shows a sensor card with sensor membranes and sampling holes positioned symmetrically about an axis perpendicular to the sensor card.
FIG. 2 shows a non-claimed sensor card with sensor membranes and sampling holes positioned in a line through the sensor card.
FIG. 3 shows an exploded view of a non-claimed sensor card.
FIG.'s 4A-F shows a sensor apparatus for use with the sensor card.
FIG. 5 shows a receiving slot cover.
FIG. 6 shows a dialysate flow path including the sensing apparatus.
FIG.'s 7A-B shows plots of the intensity of green light detected by a pH sensor membrane in the sensing apparatus as a function of pH and time.
FIG. 8 shows fitting of the detected green light intensity relative to pH of the fluid detected for a pH sensor membrane.
FIG.'s 9A-B shows the detected intensity of green light as a function of the pH of the fluid and time, as detected for a low sensitivity ammonia sensor membrane.
FIG. 10 shows fitting of the detected green light intensity relative to the ammonia concentration of the fluid as detected for a low sensitivity ammonia sensor membrane.
FIG.'s 11A-B shows the detected intensity of green light as a function of the pH of the fluid and time, as detected for a high sensitivity ammonia sensor membrane.
FIG. 12 shows fitting of the detected green light intensity relative to the ammonia concentration of the fluid as detected for a high sensitivity ammonia sensor membrane.
FIG. 13 shows fitting of the detected green light intensity relative to the ammonia concentration of the fluid as detected for a high sensitivity ammonia sensor membrane for a small range of ammonia concentrations.
FIG.'s 14A-B shows fitting of the detected green light intensity relative to pH and ammonia concentration of the fluid detected over an extended pH and ammonia range.
FIG.'s 15A-B shows the effects of uniform backlighting on the detected green light intensity for pH sensor membranes on a sensor card.
FIG.'s 16A-B shows the effects of symmetrical sensor membrane and window placement on the detected green light intensity for pH sensor membranes on a sensor card.

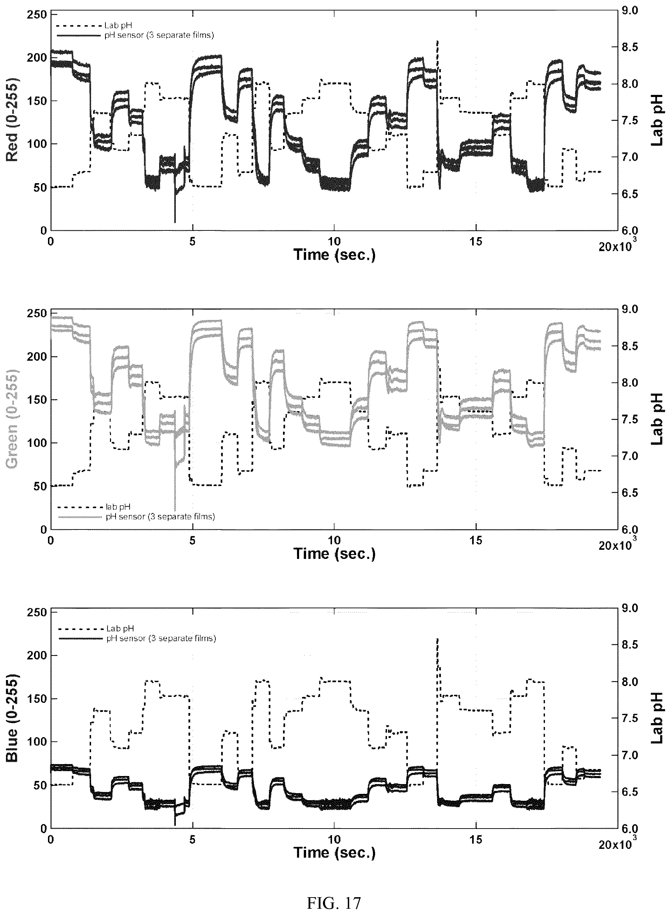

FIG. 17 shows effects of pH on the intensity of red, green, and blue light transmitted through pH sensing membranes.

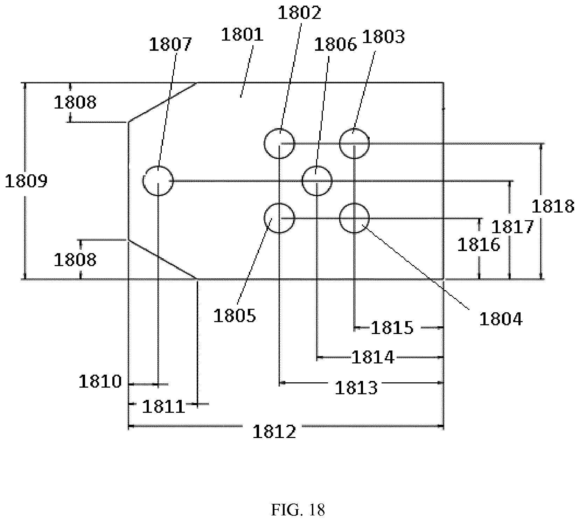

FIG. 18 is a schematic of a sensor card with four sampling holes.

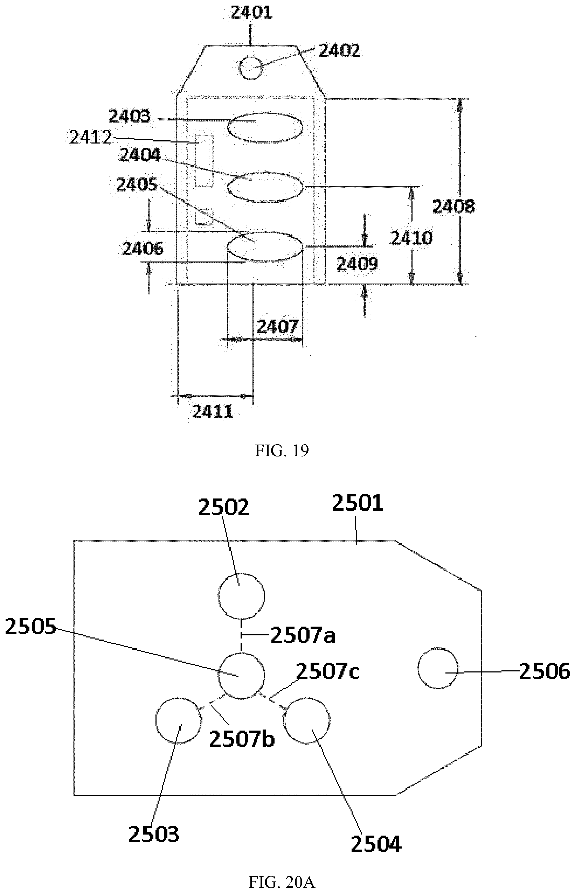

FIG. 19 is a schematic of a non-claimed sensor card.
FIG.'s 20A-C show sensor cards with circular sampling holes.
FIG.'s 21A-C show sensor cards with arced sampling holes.

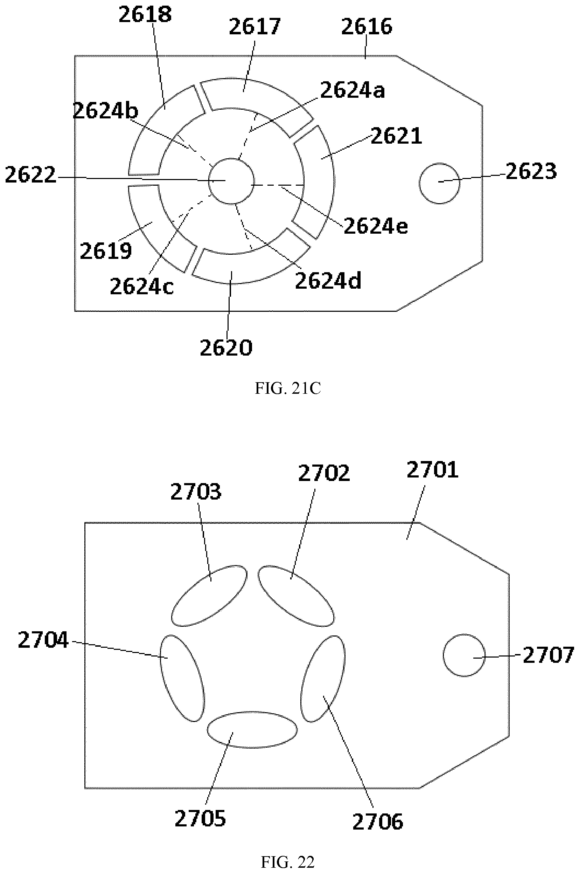

FIG. 22 shows a non-claimed sensor card with five ovoid sampling holes.

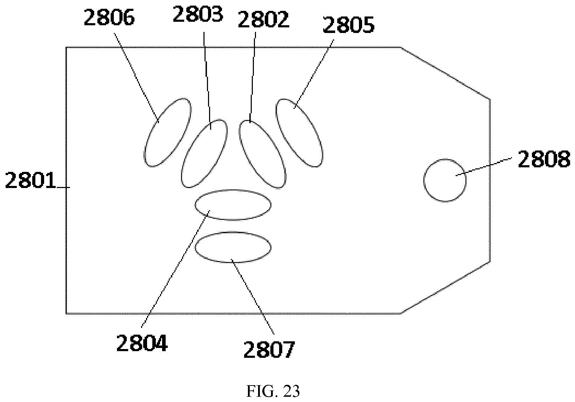

FIG. 23 shows a non-claimed sensor card with sampling holes arranged concentrically at different radii.

### DETAILED DESCRIPTION OF THE INVENTION

Unless defined otherwise, all technical and scientific terms used herein generally have the same meaning as commonly understood by one of ordinary skill in the relevant art.

The articles "a" and "an" are used herein to refer to one or to more than one (*i.e.,* to at least one) of the grammatical object of the article. By way of example, "an element" means one element or more than one element.

An "adhesive" is a component capable of forming a mechanical bond with another component to hold the two components together.

The term "aligned" refers to the relative positions of two components, wherein one component is overlaying or positioned close to the second component.

The term "ammonia concentration" refers to the amount of ammonia dissolved in a given amount of a fluid.

The term "ammonia level" refers to a concentration of ammonia (NH₃).

The term "ammonium level" refers to a concentration of ammonium cation (NH₄⁺).

The terms "arc," "arced," "arc section," and the like refer to a two-dimensional feature having a first outer edge at a circumference of a circle or curve at an outer radius and another inner edge at a circumference at a second smaller inner radius. The edges can be joined to form sections such that an arced section can sweep any number of degrees. For example, a 180° arc section will cover a semicircle, and a 90° arc section will cover a quarter of a circle.

An "axis perpendicular to a sensor card" is an imaginary line through a sensor card and at right angles to the sensor card.

The term "back side" of any material or component. In one non-limiting example, a back side can refer to a side of a sensor card facing a light emitting source when placed in a sensor apparatus.

A "bar code" is a computer readable pattern of parallel lines and spaces of variable thickness that identifies the component to which the barcode is attached.

A "camera," "photodetector," and the like is a component capable of detecting light intensity or composition to result in data, such as an image, of the light detected. The terms "camera" and "photo detector" can also refer to any type of detector including an RGB detector or spectrophotometer.

A "carrier" is a component such as a planar material that overlays or covers one or more layers. In one non-limiting example, the carrier overlays one or more sensor membranes. The terms "front carrier" or "back carrier" can refer to carriers on either side of the fluid sensor membranes on the front side and back side of the sensor card, respectively.

A "center axis" is an imaginary line through the center of a component or region. For example, a center axis can be positioned at substantially a center portion of a surface plane of a sensor card or lens and perpendicular to the surface plane.

The term "characteristic of a fluid" can refer to any physically observable property of the fluid. In one non-limiting example, the characteristic of the fluid can be the pH of the fluid or concentration of one or more solutes in the fluid

The term "circular" refers to a two-dimensional shape generally round, disk shaped, ring-shaped or annular, and having the form of a circle.

The term "color" refers to the wavelength of light reflected from or transmitted through a component or feature.

A "colorimetric material" is any material that can produce a detectable change based on one or more substances in contact with the material. The detectable change can include a visible change such as a change in color, optical transmittance, or a change in emitted fluorescent light intensity or wavelength.

The term "comprising" includes, but is not limited to, whatever follows the word "comprising." Use of the term indicates the listed elements are required or mandatory but that other elements are optional and may be present.

The term "consisting of' includes and is limited to whatever follows the phrase "consisting of." The phrase indicates the limited elements are required or mandatory and that no other elements may be present.

The term "consisting essentially of' includes whatever follows the term "consisting essentially of' and additional elements, structures, acts or features that do not affect the basic operation of the apparatus, structure or method described.

The term "cut-out" refers to a removed portion of an otherwise continuous side of a component.

The terms "detecting," "detected," or "to detect" refer to determining a state or characteristic of a system.

The terms "determining" and "determine" refer to ascertaining a particular state of a system or variable(s).

The term "dialysate" describes a fluid into or out of which solutes from a fluid to be dialyzed diffuse through a membrane.

A "dialysate flow path" is the pathway that dialysate will travel when used in normal operation for dialysis.

The term "disposed inside" refers to a first component's placement within or integral to a second component. The placement can occur by any mechanical or fixation means known to those of ordinary skill.

The term "downstream" refers to a position of a first component in a flow path relative to a second component wherein fluid will pass by the second component prior to the first component during normal operation. The first component can be said to be "downstream" of the second component, while the second component is "upstream" of the first component.

The term "equidistant" refers to two or more components or regions that are the same distance from a reference point.

The terms "fixing," to "fix," or "fixed position" refer to a position of a component that will resist inadvertent movement.

The terms "flow," "flowing," and the like refer to a stream of gas, liquid, or combinations thereof moving, issuing, or circulating with a continual change of place among the constituent particles. As used in the phrase "flowing a fluid," the term refers to a stream of liquid.

A "fluid" is a liquid substance optionally having a combination of gas and liquid phases in the fluid. Notably, a liquid, as used herein, can therefore also have a mixture of gas and liquid phases of matter.

The term "fluid contact" refers to a component that, in use, will touch or come into contact with a fluid.

A "fluid sensor membrane" is a substrate with an embedded dye. The embedded dye can change color, change an amount or wavelength of transmitted light, and/or change an amount or wavelength of fluorescent light in response to a fluid characteristic, such as a particular solute concentration or pH, of a fluid contacting the sensor membrane. The fluid sensor membrane can also detect gas and combinations of gases dissolved in the fluid. Although the term "fluid" is used in "fluid sensor membrane," the "fluid sensor membrane" is not limited to use with just fluids, but can also be used for gases and gases dissolved in fluid.

The term "front side" refers to a side of any surface or material. In one non-limiting example, a "front side of a sensor card" can face a camera when placed in a sensor apparatus.

A "high sensitivity ammonia sensor membrane" is an ammonia sensor membrane capable of detecting changes in ammonia concentration less than 2 ppm ammonia.

The term "hole" refers to an opening from one side to another side of a component.

The term "intensity" refers to the amplitude of a light wave.

The term "interposed" refers to a component being positioned between two other components.

An "LED array" is any configuration of light emitting diodes. In one non-limiting example, the LED array is a circular or consistently spaced placement of individual LED lights. The term "array," as used herein, is not intended to be limited to any particular configuration, but conveys a regularized or uniform positioning of individual LED lights. The term "LED array" is not limited to any color or colors of LEDs or any particular placement of LEDs.

The term "lens" refers to a glass or transparent component for receiving light. In reference to a camera or photodetector, the term can refer to a transparent component of the photodetector or camera for receiving light rays.

A "light emitting source," "light emitter," "photo emitter," or the like, is any component capable of emitting light at any wavelength including visible, infrared, or ultraviolet light.

A "low sensitivity ammonia sensor membrane" is a substrate with an embedded dye, wherein the dye changes colors in response to the ammonia concentration of a fluid, and the dye can detect changes in ammonia concentration over a range of between 2-20 ppm ammonia.

The term "non-reflective" refers to a material or color that absorbs substantially all visible or ultraviolet light.

The term "opposite side" refers to a first side of a component or reference that faces or is positioned in a direction 180° away from a second side of the component.

The terms "opposing" and "opposedly positioned" refer to relative positions of two or more components wherein the two or more components are positioned on opposite sides of a reference.

The term "optically observable change" refers to any change in a component that can be detected based on an intensity or wavelength of light transmitted through or reflected from the component. The change can be a physical change such as color, deformation, or any other change in physical property.

The term "overlaying" refers to a first component being positioned on top of, or covering, a second component.

The term "ovoid" refers to a two-dimensional shape having rounded ends and a slightly elongated shape.

A "perimeter of a circle" refers to the portion of a circle around the circumference of the circle.

The term "perpendicular center axis" refers to a line positioned at a center of a surface place and at a right angle to the surface plane.

The term "pH" refers to the negative log of the H⁺ concentration in a fluid when stated in moles of H⁺ per liter of fluid volume.

A "pH sensor membrane" is a substrate with an embedded dye, wherein the dye changes colors in response to the pH of a fluid.

"Polypropylene" is a polymer made from the polymerization of propylene and having a chemical structure of carbon atoms wherein every other carbon atom is bound to a methyl group.

The term "positioned" or "position" refers to a physical location of a component, feature, or structure.

The term "positioned concentrically" refers to the relative position of at least two components, wherein each component is on a perimeter of a circle around a reference point.

The term "positioned symmetrically" refers to the relative position of at least two components, wherein each component is positioned at the same angle and distance from a reference point.

A "pressure equalizing hole" is a hole positioned through a component for equilibrating pressure on each side of the component on which the hole is formed.

The terms "pumping," "pumped," or to "pump" refer moving a fluid, gas, or combination thereof, with a pump.

The term "radius" refers to the distance between a center of a circle and a perimeter of the circle.

The term "radius about a center axis" refers to a distance between a perimeter of a circle and a center axis of a component or system.

The term "rectangular" refers to a two-dimensional shape having four edges and four angles. This description is not intended to limit the size and dimensions of the described components, and may therefore encompass components having corners with angles greater than or less than ninety degrees, and with edges of differing lengths with respect to each other.

A "reference sampling hole" is a sampling hole positioned over a reference sensing region.

A "reference sensing region" is a region on a surface having a color that does not change with respect to a characteristic of a fluid.

A "sampling hole" is a hole in a portion of a surface through which fluid and light can pass to contact a sensing membrane. In one non-limiting example, the sensing membrane can be a fluid sensing membrane.

The term "securedly fastening," "securely fastening," "secured fastening," and the like refer to fixing one component to another component. In one non-limiting example, a sensor card can be securely fastened within a sensor apparatus such that the sensor card resists inadvertent movement.

A "sensor apparatus" refers to an apparatus adapted for use with a sensor card, described herein, through which fluid can be pumped to contact the sensor membranes of the sensor card and determine a characteristic of the fluid.

The term "sensor card" refers to a rigid and/or planar component having at least one sensing membrane or sensing material of any kind disposed on, inside or integral to the "sensor card." The sensing membrane or material contained inside the sensor card can contact a fluid, and produce a detectable change in response to a fluid characteristic of the fluid.

The term "sorbent cartridge" refers to a cartridge containing one or more sorbent materials for removing specific solutes from solution. The term "sorbent cartridge" does not require the contents in the cartridge be sorbent based, and the contents of the sorbent cartridge can be any contents that can remove solutes from a dialysate. The sorbent cartridge may include any suitable amount of one or more sorbent materials. In certain instances, the term "sorbent cartridge" refers to a cartridge which includes one or more sorbent materials besides one or more other materials capable of removing solutes from dialysate. "Sorbent cartridge" can include configurations where at least some materials in the cartridge do not act by mechanisms of adsorption or absorption.

The term "substantially aligned" refers to alignment to a large extent of one or more lines, surfaces, axis, with or to another. For example, two lines can be substantially aligned with some deviation from each other such that the essential characteristics of the alignment are not lost.

The term "tapered edge" refers to a component with at least one edge set at a different angle from a connecting edge.

The term "transmitting light" or to "transmit light" refers to the passage of light from one side of a component through the component to an opposite side of the component.

The term "triangular" refers to a two-dimensional shape having three sides.

The term "trigger" means to cause some action or effect.

The term "uniformly transmitting" or to "uniformly transmit" refer to distributing a quantity of the energy of the light emitted per second evenly onto or through a surface.

The term "upstream" refers to a position of a first component in a flow path relative to a second component wherein fluid will pass by the first component prior to the second component during normal operation. The first component can be said to be "upstream" of the second component, while the second component is "downstream" of the first component.

### Flow Assembly Card

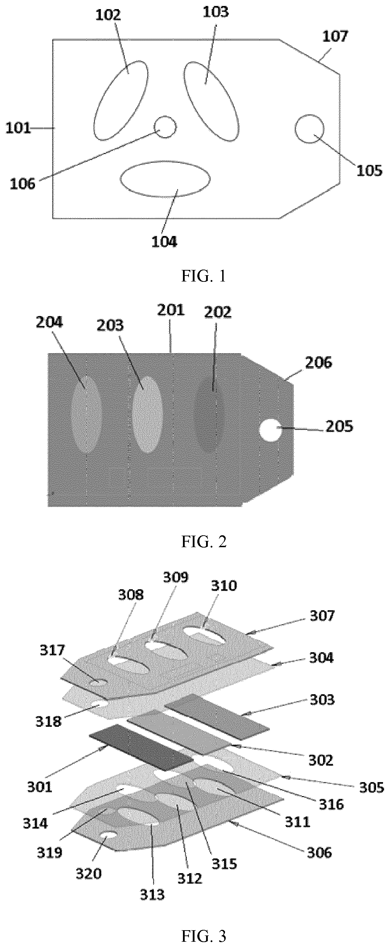

FIG. 1 illustrates a non-limiting embodiment of a flow assembly card for sensing fluid characteristics of a fluid. The sensor card **101** can include one or more fluid sensor membranes or colorimetric materials disposed inside the sensor card **101.** The sensor card in FIG. 1 has a first fluid sensor membrane **102,** a second fluid sensor membrane **103,** and a third fluid sensor membrane **104.** The fluid sensor membranes have a sensor dye embedded in a substrate. The sensor dye produces an optically observable change, such as a change in color, triggered in response to a fluid characteristic of the fluid, including pH or the presence of ions in solution, such as ammonia. In addition to color changes, the optically observable change can be changes to the intensity of light transmitted through the sensor dye, a deformation in a shape of the sensor dye, or any other change that is optically observable in either color or gray scale. The fluid sensor membranes can include any combination of pH sensor membranes, high sensitivity ammonia sensor membranes, and low sensitivity ammonia sensor membranes. The pH sensor membrane can detect pH in a range of 6.8 to 7.8, the high sensitivity ammonia sensor membrane can detect ammonia in a range of 1 ppm to 2 ppm, and the low sensitivity ammonia sensor membrane can detect ammonia in a range of 1 ppm to 20 ppm. Although illustrated as three fluid sensor membranes in FIG. 1, the sensor card can have any number of fluid sensor membranes, including 1, 2, 3, 4, 5, 6, or more fluid sensor membranes. Fluid can contact each of the fluid sensor membranes through sampling holes in the sensor card. Depending on the pH and ammonia concentration of the fluid, the fluid sensor membranes will change color, optical transmittance, or change emitted fluorescent light intensity or wavelength, which can then be detected to determine the pH and/or ammonia concentration of the fluid. However, the sensor card is not limited to pH and ammonia sensing membranes, and can include any colorimetric material producing a detectable change in response to the presence of solutes, such as ions, in a solution or other parameter of a fluid. In general, the colorimetric material can produce any visible change such as a change in color or optical transmittance, or a change in emitted fluorescent light intensity or wavelength, wherein the visible change is detected by the photodetector or camera of the present invention. Non-limiting examples of colorimetric materials that can be embedded in a sensing membrane include bromothymol blue for the detection of antifreeze or other substances, lead acetate for the detection of sulfides, glucose oxidase for the detection of glucose, benzidine-type chromogens for the detection of chlorine, or any other colorimetric materials known in the art. Additional materials that can be included in the sensing membranes include ACUSTRIP 711254 for detection of antifreeze coolant in automatic transmission fluid, ACU987600 for detection of ethanol in fuel, Acustrip Metals Test® for detection of wear metals in fluid, and the Acustrip 84050 mold test for the presence of mold in a fluid, each available from Acustrip®, a New Jersey corporation. Other non-limiting colorimetric materials include materials for testing alkalinity, aluminum, ammonium, calcium, carbonate, chloride, chlorine, chlorine dioxide, chromate, color, copper, cyanide, fluoride, formaldehyde, hydrazine, iron, magnesium, manganese, nickel, nitrate, nitrite, oxygen, ozone, pH, phosphate, residual hardness, silicate, sulfate, sulfide, sulfite, total hardness, urea, and zinc, each available from EMD Millipore, a Massachusetts corporation. The sensor card can also include only a pH sensing membrane, only an ammonia sensing membrane, or a sensing membrane having any type of colorimetric material.

As illustrated in FIG. 1, the sensor card **101** can have an axis perpendicular to the sensor card **101** through point **106.** The fluid sensor membranes **102-104,** or other colorimetric material, and sampling holes can be arranged around the center axis of the sensor card **101,** on a perimeter of a circle having a radius about the axis perpendicular to the sensor card **101** through point **106.** The axis perpendicular to the sensor card **101** through point **106** of the sensor card **101** can be substantially aligned to a perpendicular center axis of a lens of a photo-detector or a camera positioned to capture an image of the sensor card so that the sensor can be aligned to the camera or photodetector. The radius can extend from the axis perpendicular to the sensor card **101,** with the fluid sensor membranes **102-104** positioned on the perimeter of the circle. As described, a light emitting source and camera can detect the color of the fluid sensor membranes **102-104.** The light source can transmit light through the sensor card **101,** and the camera can detect the transmitted light. Spherical aberration in the images produced by the camera due to differing distances of the fluid sensor membranes from the center axis of the camera can degrade the accuracy of the sensor. Having the fluid sensor membranes **102-104** positioned symmetrically on a perimeter of a circle with a radius extending from the center **106** can reduce the spherical aberration by placing the fluid sensor membranes **102-104** and sampling holes equidistant to the center axis of the sensor card **101.** The fluid sensor membranes and sampling holes can alternatively be positioned concentrically around the center axis of the sensor card, including at differing radii from the center axis.

As illustrated in FIG. 1, the sensor card **101** can include a pressure equalizing hole **105** through the sensor card. Because fluid can be flowed across both sides of the sensor card **101,** the fluid pressure on either side may increase or decrease. The pressure equalizing hole **105** allows for fluid to move from one side of the sensor card **101** to the other, equalizing the pressure on either side of the sensor card **101.**

The sensor card can be constructed in any shape. As illustrated in FIG. 1, the sensor card **101** can be substantially rectangular. However, any shape can be used for the sensor card, including rectangular, ovoid, circular, triangular, arced, and combinations thereof. The sensor card **101** can also have at least one tapered edge **107.** The tapered edge **107** can be inserted into a bevel of the sensor apparatus, further fastening the sensor card **101** in place. The tapered edge **107** can be sized and shaped to correspond to any suitable complimentary size and shape of the bevel in the sensor apparatus.

The sensor card can also include a bar code (not shown) fixed on a surface of the sensor card. A bar code identifies the sensor card. The usage of the sensor card can be tracked by reading the bar code, and counterfeit sensor cards can be identified. Patient or machine information and other patient or machine specific data can be stored on the bar code. The bar code can also include information on calibration of specific lots of sensor membranes contained in an individual card.

In one embodiment, the fluid sensor apparatus of the invention can detect pH changes of ±0.2 pH units within 10 minutes with a reliability of 95% and confidence of 95% in a pH range of around 6.8 to 7.8. The fluid sensor can also detect pH changes at any one of ±0.25 pH units, ±0.3 pH units, ±0.15 pH units, or ±0.1 pH units with reliability of >75% and confidence of >75%. The fluid sensor apparatus of the invention can also measure pH changes with an accuracy of ±0.1 pH units with a reliability of 95% and confidence of 95% in a pH range of around 6.8 to 7.8. Further, the fluid sensor can measure pH changes with an accuracy of any one of ±0.05 pH units, ±0.15 pH units, ±0.2 pH units, or ±0.3 pH units with reliability of >75% and confidence of >75%. The pH detection range is dependent upon the pH dye used, and can be altered by changing the pH sensitive dye. At a total ammonia concentration range of 1 to 20 ppm, the fluid sensor apparatus of the invention can detect ±1 ppm total ammonia changes within 10 minutes with a reliability of 95% and confidence of 95% in a pH range of around 6.8 to 7.8. The fluid sensor apparatus can also detect total ammonia at any one of ±0.5 ppm, ±1.5 ppm, ±2.0 ppm, or ±2.5 ppm with reliability of >75% and confidence of >75%. The ammonia detection range is dependent upon the ammonia sensitive dye used, and can be altered by changing the ammonia sensitive dye. At a total ammonia concentration range of 1 to 5 ppm, the fluid sensor apparatus of the invention can measure total ammonia concentration with an accuracy of ±0.2 ppm total ammonia changes within 10 minutes with a reliability of 95% and confidence of 95% in a pH range of around 6.8 to 7.8. Alternatively, the fluid sensor can measure total ammonia concentration with an accuracy at any one of ±0.5 ppm, ±1.5 ppm, ±2.0 ppm, or ±2.5 ppm with reliability of >75% and confidence of >75%. The sensor card is not limited to ammonia and pH detection, and can detect any fluid characteristic or concentration of ions or other solutes in solution of a liquid or gaseous fluid. Any colorimetric material can be included in the sensing membranes for detection of any substance.

FIG. 2 illustrates a non-claimed sensor card **201** with three fluid sensor membranes **202-204** and sampling holes. The sensor card of FIG. 2 has the fluid sensor membranes **202-204** arranged in a row as opposed to having the fluid sensor membranes equidistant from an axis perpendicular to the sensor card. With any arrangement of fluid sensor membranes, the sensor card **201** can include a pressure equalizing hole **205** for equalizing the fluid pressure on either side of the sensor card **201** in a sensor apparatus. The sensor card **201** can include one or more tapered edge **206,** which can be inserted into a bevel on the sensor apparatus to secure the sensor card in place.

In FIG.'s 1-2, the fluid sensor membranes are shown as substantially ovoid. However, the sampling holes and/or fluid sensor membranes can be any shape, including rectangular, ovoid, circular, triangular, arced, and combinations thereof. Further, the fluid sensor membranes and sampling holes can be arranged in any fashion on the sensor card.

FIG. 3 illustrates an exploded view of a non-claimed sensor card. The sensor card can include one or more fluid sensor membranes disposed inside the sensor card, including a high sensitivity ammonia sensor membrane **301,** a low sensitivity ammonia sensor membrane **302,** and a pH sensor membrane **303,** or any combination thereof. The pH and/or ammonia sensor membranes can be placed between two adhesive layers **304** and **305** interposed between a front carrier **307** and a back carrier **306,** which overlay a front side and back side of the sensor card, respectively. The adhesive layers **304** and **305** can affix the fluid sensor membranes to the front carrier **307** and back carrier **306.** The adhesive layers and front and back carriers can include sampling holes to allow fluid to contact the fluid sensor membranes. Sampling holes **308, 309,** and **310** in front carrier **307** allow transmitting light and fluid through the front carrier **307.** Sampling holes **311, 312,** and **313** allow light and fluid to pass through back carrier **306.** The sampling holes **308, 309,** and **310** in front carrier **307** and the sampling holes **311, 312,** and **313** in back carrier **306** are opposedly positioned on opposite sides of the sensor card. Cut-outs **314, 315,** and **316** allow light and fluid to pass through adhesive layer **305.** Although not shown in FIG. 3, adhesive layer **304** also has cut-outs aligned over the fluid sensor membranes and sampling holes. As described, the sampling holes and cut-outs can be any shape, and need not be the same shape as the fluid sensor membranes. A pressure equalizing hole can penetrate each layer of the sensor card, shown as pressure equalizing hole **317** in front carrier **307,** pressure equalizing hole **318** in adhesive **304,** pressure equalizing hole **319** in adhesive **305,** and pressure equalizing hole **320** in back carrier **306.** As described the pressure equalizing hole can function to equalize the pressure on either side of the sensor card during use.

In a single-sided embodiment, the sensor card of FIG. 3 can have just one of the two carriers disposed thereon. That is, only one of either the front or back carrier can be positioned with an adhesive layer, and the sensor membranes described herein. In the single-sided embodiment, the sensor card has the one fluid sensor membrane positioned underneath the single carrier side overlaying one side of the fluid sensor membrane; and also one sampling hole positioned on the carrier aligned over the one side of the fluid sensor membrane wherein the fluid sensor membrane comprises a colorimetric material. The carrier material in the single-sided embodiment can be sufficiently darkened or have a thickness to be substantially opaque to prevent light transmission through the single-sided carrier material to avoid interfering with photo-detection on the camera side.

The front and/or back carriers of the sensor card can be made of any material known in the art, including polypropylene, polyvinyl chloride, or any rigid, optically opaque plastic, including dyed polytetrafluoroethylene (PTFE), ethylene tetrafluoroethylene (ETFE), polyvinylidene difluoride (PVDF), fluorinated ethylene propylene (FEP), polyethylene (PE), polyimide (PI), or polyetheretherketone (PEEK). The fluid sensor membranes or colorimetric materials can have a dye embedded in or chemically bound to a substrate, and a change in color of the dye, a change in the intensity of light transmitted through the dye, or a change in the fluorescent light from the dye, can be triggered in response to the pH or ammonia concentration of a fluid, or the presence or concentration of any solutes or ions in solution. The substrate can be any substrate known in the art capable of allowing gaseous ammonia through the substrate to contact the embedded dye, including polytetrafluoroethylene (PTFE), polyvinylidene difluoride (PVDF) and other fluorinated, hydrophobic polymers such as fluorinated ethylene propylene (FEP) and ethylene tetrafluoroethylene (ETFE). The gaseous ammonia penetrates the substrate and contacts the dye, altering the color of the dye. The ammonia sensitive dye can be any dye capable of producing an optically observable change, such as changing color, in response to the ammonia concentration, including bromophenol-blue, bromocreosol green, thymol blue, methyl crystal purple, chlorophenol, free-base porphyrins, Tetraphenylporphyrin (H2TPP), and combinations thereof. The pH sensitive dye can include Bromocresol Purple, Bromothymol Blue, Phenol Red, Thymol Blue, or combinations thereof.

The sensor membranes or colorimetric materials can be any material sensitive to a component of the fluid in the fluid path to be sensed. In general, the sensor membrane or colorimetric material has a property reacting to a fluid component that changes an optical parameter depending on the concentration of the component in the fluid. The optical parameter can be any one of color, reflectivity, fluorescence, adsorption, or any other parameter capable of being optically detected. In a preferred embodiment, the sensor membrane or colorimetric material changes color in relationship to changes in the solute concentration of the measured fluid component. The term solute concentration refers to the amount of a first substance, such as ions or other solutes, dissolved in a second substance. For example, the membrane can change color in a first direction along a color spectrum as the solute concentration of the component in the fluid increases, and along a second direction as the solute concentration of the component decreases. The color change of the membrane can be continuous and reversible in response to the component concentration. In the case of an ammonia sensor membrane, a dye can be embedded in a substrate, wherein the dye changes colors in response to an ammonia concentration of a fluid.

The sensor card can have any dimensions usable in a sensor apparatus. The sensor card can have any thickness. Above a certain value of thickness relative to the window size, the edges of the card will cast shadows that may interfere with detection of the transmitted light. In a preferred embodiment, the thickness of the sensor card is limited to less than 50% of the smallest dimension of the film window, or between 0.5 and 3 mm for a sampling hole having a smallest dimension of 6 mm. The sensor card can be any length, including between 16 and 48 mm. The sensor card can have any width, including between 10 and 30 mm. The front and back carriers of the sensor card can be between 0.1 and 0.3 mm thick. The adhesive layers can be between 0.08 and 0.25 mm thick. The ammonia sensor membranes can be between 0.06 and 0.19 mm thick. Experiments have shown that a pH sensor membrane of the same thickness allows more light than desired to be transmitted through the sampling holes. As such, the pH sensor membrane can be thicker, including between 0.12 and 0.38 mm. Each sampling hole can have any diameter that will fit in the sensor card, including between 5 and 15 mm.

In addition to the fluid sensor membranes or colorimetric material illustrated in FIG. 3, the sensor card can also include a reference sensing region (not shown in FIG. 3). The reference sensing region can be a region of the sensor card colored in a solid color and can include a reference sampling hole positioned over the reference sensing region. As described, detection of green light transmitted through the sensor card can provide the most accurate sensing of pH and/or ammonia. The reference sensing region can be colored green, and used by the processor as a reference in determining the intensity of green light transmitted through the sensor card. The reference sensing region provides a reference against which the changes in color or intensity from the sensing membranes can be compared. The reference sensing region allows monitoring and control for any changes in the optical path of light due to dirty or scratched windows or variable light intensity from the light source. However, any light color can be used such as red and blue for sensing pH and/or ammonia. The definitions of red, green, and blue light can be based on the camera operating software, or can be more specific. A spectrophotometer, which measures the wavelength and intensity of the transmitted light can also work and would be more specific on the wavelengths of light detected. The reference sensing region can be positioned at any point on the sensor card, including on the axis perpendicular to the sensor card.

FIG. 18 illustrates a non-limiting schematic of a sensor card **1801.** The sensor card **1801** can include four sampling holes **1802, 1803, 1804,** and **1805,** as well as reference sensing region **1806.** Alternatively, each of **1802, 1803, 1804, 1805,** and **1806** can each be sampling holes, with a separate reference sensing region optionally provided. Further, any number of sampling holes and reference sensing regions can be included. For example, the sensor card **1801** can have two reference sensing regions and three sampling holes, three reference sensing regions and two sampling holes, or four reference sensing regions and one sampling hole. The sampling holes can overlay fluid sensor membranes or other colorimetric materials disposed inside the sensor card **1801,** including a high sensitivity ammonia sensor membrane, a low sensitivity ammonia sensor membrane, and a pH sensor membrane, or any combination thereof. One of skill in the art will understand that the order of the fluid sensor membranes can be changed. Pressure equalizing hole **1807** can equalize the fluid pressure on either side of the sensor card during use. The sensor card can be any length, shown as distance **1812,** including between 16 and 48 mm. The sensor card can be any width, shown as distance **1809,** including between 10 and 30 mm. The sensor card **1801** can include at least one tapered edge, tapering inwardly along a side of the sensor card. The tapered edge can begin any distance from the end of the sensor card **1801,** shown as distance **1811,** including between 3.5 and 10.5 mm from the end of the sensor card **1801.** The tapered edge can taper to any degree, shown as distance **1808,** including between 2.0 and 6.0 mm from the side of the sensor card **1801.** The pressure equalizing hole **1807** can be any distance from the end of the sensor card **1801,** shown as distance **1810,** including between 1.5 and 4.5 mm from the edge of the sensor card **1801.**

The sensor card **1801** can include each of the sampling holes **1802-1805** concentrically arranged about an axis perpendicular to the sensor card **1801,** with the reference sensing region **1806** at the axis. The sampling holes **1802** and **1805** can be any distance from the bottom of the sensor card, shown as distance **1813,** including between 25 and 8.0 mm. The reference sensing region **1806** can be any distance from the bottom of the sensor card, shown as distance **1814,** including between 19 and 6.5 mm. The sampling holes **1803** and **1804** can be any distance from the bottom of the sensor card, shown as distance **1813,** including between 4.5 and 13.6 mm. Sampling holes **1804** and **1805** can be positioned any distance from the side of the sensor card, shown as distance **1816,** including between 3.1 and 9.3 mm. The reference sensing region **1806** can be positioned any distance from the side of the sensor card, shown as distance **1817,** including between 5.0 and 15.0 mm. Sampling holes **1802** and **1803** can be positioned any distance from the side of the sensor card, shown as distance **1818,** including between 21 and 6.9 mm.

FIG. 19 illustrates a non-limiting schematic of a non-claimed sensor card **2401.** The sensor card **2401** can include three sampling holes **2403, 2404,** and **2405.** The sampling holes can overlay fluid sensor membranes disposed inside the sensor card **2401,** including a high sensitivity ammonia sensor membrane, a low sensitivity ammonia sensor membrane, and a pH sensor membrane, or any combination thereof. One of skill in the art will understand that the order of the fluid sensor membranes can be changed. Pressure equalizing hole **2402** can equalize the fluid pressure on either side of the sensor card during use. The sensor card can be any length, shown as distance **2408,** including between 16 and 48 mm. Each ovoid sampling hole can have a small diameter of any length, shown as distance **2406,** including between 2 and 6 mm. Each ovoid sampling hole can have a large diameter of any length, shown as distance **2407,** including between 5 and 15 mm. The distance between a bottom edge of the sensor card **101** and the center of the first sampling hole **2405** can be any length, shown as distance **2409,** including between 2.5 and 7.5 mm. The distance between the bottom edge of the sensor card **101** and the center of the second sampling hole **2404** can be any length, shown as distance **2410,** including between 6.5 and 19.5 mm. As described, placing the sampling windows in areas of uniform illumination within the sensor apparatus can increase accuracy of the sensor. As such, the distances **2409** and **2410** can be varied to place each sampling window in an area of uniform illumination, which causes the light or the light energy to be uniformly transmitted to the sampling holes. The sampling holes **2403-2405** can be placed on axis perpendicular to the sensor card **2401,** or offset from the axis. The sensor card **2401** can have any width, including between 10 and 30 mm. Distance **2411** is the distance from the edge of the sensor card **2401** to the center of the sensor card **2401** and can be between 5 and 15 mm. A barcode **2412** or other identification component can be included on the sensor card **2401** for identification and tracking of the sensor card **2401.** An LED or other light source can be included in the sensor apparatus on the same side of the sensor card as the camera to illuminate the barcode **2412** for reading by the camera.

FIG.'s 1-3 illustrate sensor cards with ovoid sampling windows. As described, the sampling windows can be in any shape, including circular, rectangular, triangular, or arced. FIG.'s 20A-C illustrate sensor cards with substantially circular sampling holes. FIG. 20A illustrates a sensor card **2501** with three sampling holes **2502, 2503,** and **2504.** Each sampling hole **2502-2504** can overlay a fluid sensor membrane. As illustrated in FIG. 20A, the sampling holes **2502-2504** can be positioned symmetrically around axis perpendicular to the sensor card **2501** and equidistant from the axis, as shown by dashed lines **2507a, 2507b,** and **2507c.** However, any arrangement of sampling holes can be used. With any arrangement of sampling holes, the sensor card **2501** can include a pressure equalizing hole **2506** for equalizing pressure on each side of the sensor card **2501** in a sensor apparatus. An optional reference sensing region **2505** can be included at any location on the sensor card **2501,** including on the center axis of the sensor card **2501,** as illustrated in FIG. 20A.

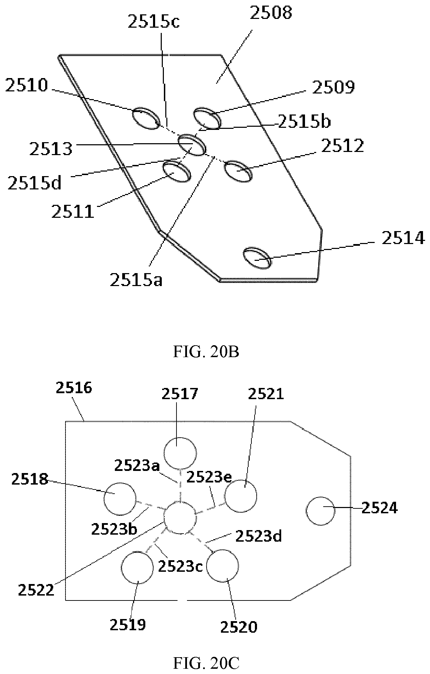

FIG. 20B illustrates a sensor card **2508** with four sampling holes **2509, 2510, 2511,** and **2512** positioned symmetrically around axis perpendicular to the sensor card **2508** and equidistant from the axis, as shown by dashed lines **2515a, 2515b, 2515c,** and **2515d.** Optional reference sensing region **2513** can be included at any location. A pressure equalizing hole **2514** can be included equalize the fluid pressure on each side of the sensor card **2508** in the sensor apparatus. FIG. 20C illustrates a sensor card **2516** with five sampling holes **2517, 2518, 2519, 2520,** and **2521** positioned symmetrically around an axis perpendicular to the sensor card **2516** and equidistant from the axis, as shown by dashed lines **2523a, 2523b, 2523c, 2523d,** and **2523e.** Optional reference sensing region **2522** can be included at any location. A pressure equalizing hole **2524** can be included to equalize the fluid pressure on either side of the sensor card **2516** in the sensor apparatus. Any number of sampling holes can be included in any sensor card, including 2, 3, 4, 5, 6, 7, or more.

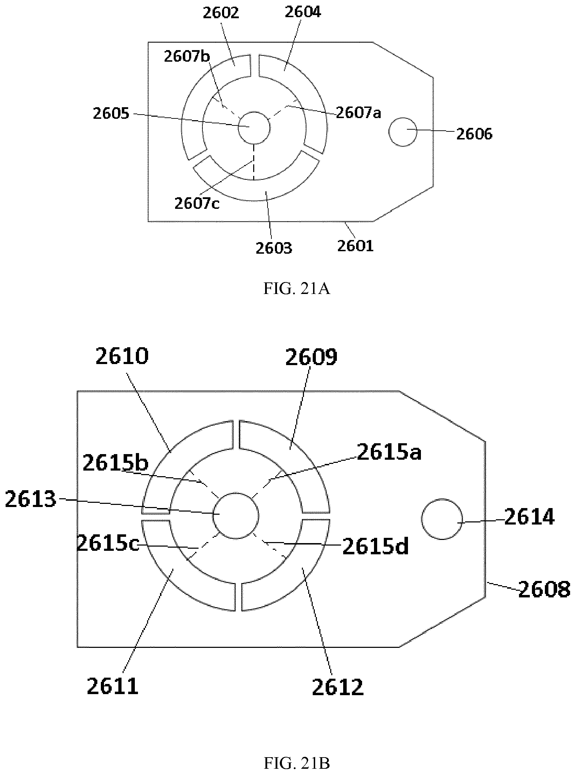

FIG.'s 21A-C illustrate a sensor card with arced sampling holes. FIG. 21A illustrates a sensor card **2601** with three sampling holes **2602, 2603,** and **2604.** Each sampling hole **2602-2604** can overlay a fluid sensor membrane. As illustrated in FIG. 21A, the sampling holes **2602-2604** can be positioned symmetrically about an axis perpendicular to the sensor card **2601** and equidistant from the axis, as shown by dashed lines **2607a, 2607b,** and **2607c.** However, any arrangement of sampling holes can be used. With any arrangement of sampling holes, the sensor card **2601** can include a pressure equalizing hole **2606** for equalizing the fluid pressure on either side of the sensor card **2601** in a sensor apparatus. An optional reference sensing region **2605** can be included at any location on the sensor card **2601,** including on the center axis of the sensor card **2601,** as illustrated in FIG. 21A. FIG. 21B illustrates a sensor card **2608** with four sampling holes **2609, 2610, 2611,** and **2612** positioned symmetrically about an axis perpendicular to the sensor card **2608** and equidistant from the axis, as shown by dashed lines **2615a, 2615b, 2615c,** and **2615d.** Optional reference sensing region **2613** can be included at any location. A pressure equalizing hole **2614** can be included to equalize the pressure on either side of the sensor card **2608** during use. FIG. 21C illustrates a sensor card **2616** with five sampling holes **2617, 2618, 2619, 2620,** and **2621** positioned symmetrically around an axis perpendicular to the sensor card **2616** and equidistant from the axis, as shown by dashed lines **2624a, 2624b, 2624c, 2624e,** and **2624e.** Optional reference sensing region **2622** can be included at any location. A pressure equalizing hole **2623** can be included to equalize the fluid pressure on either side of the sensor card during use.

As described, the sensor card can include any number of sampling holes and fluid sensor membranes. FIG. 22 illustrates a non-claimed sensor card **2701** with five sampling holes **2702, 2703, 2704, 2705,** and **2706** positioned symmetrically about an axis perpendicular to the sensor card **2701.** One of skill in the art will understand that any number of sampling holes and sensor membranes can be included in the sensor card, including 1, 2, 3, 4, 5, 6, or more sampling holes and sensor membranes of any type. Multiple sensing membranes of the same type can provide redundancy and further accuracy. In addition to a pH sensor membrane, a low sensitivity ammonia sensor membrane, and a high sensitivity ammonia sensor membrane, additional fluid sensor membranes of varying sensitivities can be included. With any number of sampling holes and fluid sensor membranes, pressure equalizing hole **2707** can be included to equalize the pressure on either side of the sensor card **2701** during use.

FIG. 23 illustrates a non-claimed sensor card **2801** with six sampling holes positioned concentrically about an axis perpendicular to the sensor card **2801** at different radii. Sampling holes **2802, 2803,** and **2804** are positioned concentrically about the axis perpendicular to the sensor card **2801** at a first radii, while sampling holes **2805, 2806,** and **2807** are positioned concentrically about the axis perpendicular to the sensor card **2801** at a second radii. Any number of radii can be used for position of the sampling holes, including 1, 2, 3, 4, or more. Pressure equalizing hole **2808** equalizes the fluid pressure on either side of sensor card **2801** during use.

The pressure equalizing hole can be placed in any location on the sensor cards, including on a side edge of the sensor card, on a bottom edge of the sensor card, or in any other location. One of skill in the art will understand that any combination of sensor card shape, sampling hole number, sampling hole arrangement, and pressure equalizing hole location can be used. A reference sensing region can be included in any sensor card at any location. The sensor card can be any color. In a preferred embodiment, one or more surfaces of the sensor card can be made of a non-reflective material, or colored in a non-reflective color, such as black, which can improve the accuracy of the sensor, such as the front and back carrier

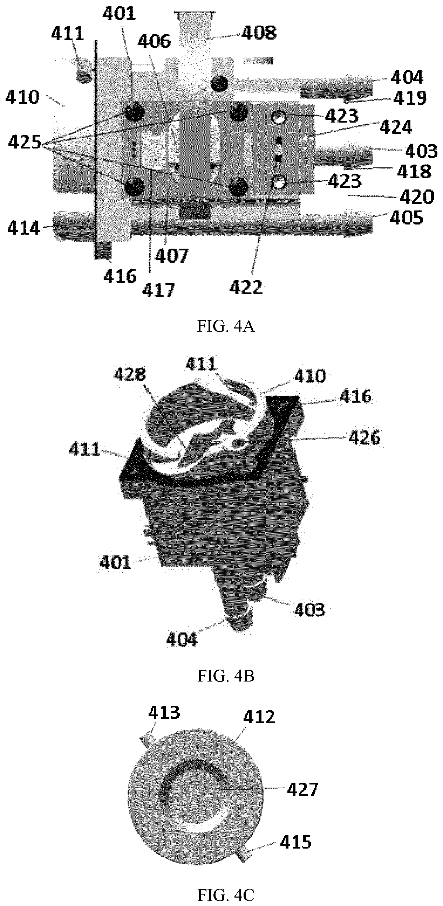

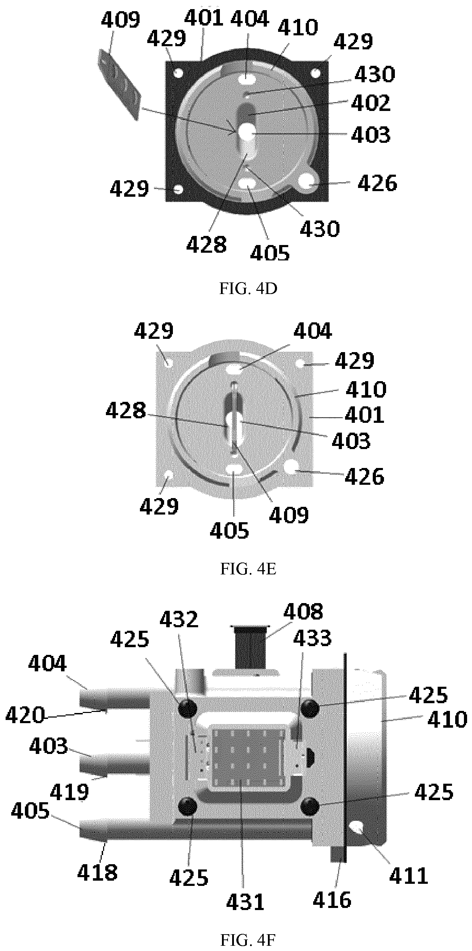

FIG.'s 4A-F illustrate a non-limiting embodiment of a sensor apparatus usable with the described sensor cards. FIG. 4A illustrates a side view of the sensor apparatus **401;** FIG. 4B illustrates a front view of the sensor apparatus **401;** FIG. 4C illustrates a receiving slot cover **412** for the sensor apparatus **401;** FIG. 4D illustrates a cut-away portion of the sensor apparatus **401** at a specified depth and a sensor card **409** being inserted into a receiving slot **402** of the sensor apparatus **401;** FIG. 4E illustrates a front view of the sensor apparatus **401,** with the sensor card **409** inserted; and FIG. 4F illustrates a side view of the sensor apparatus **401.**

As shown in FIG. 4D, the sensor apparatus **401** has a receiving slot **402** traversing a sampling chamber **428** along an axis. A removable sensor card **409,** as described herein, can be inserted into the receiving slot **402** as illustrated in FIG.'s 4D and 4E to a specified depth of the sensor apparatus **401.** Indentations **430** on either side of the sampling chamber **428** at the depth of the sensor apparatus **401** as shown in FIG. 4D, can receive an edge of the sensor card **409,** to seat or fasten the sensor card **409** in place. At a higher depth of the sensor apparatus **401,** a groove can be formed appurtenant to a sidewall of the sampling chamber **428** to receive a side edge of the sensor card **409.** An edge of the sensor card **409** can be securely positioned in the sampling chamber **428** at a specified location or orientation with respect to a light emitting source and/or photo detector. Alternatively, one or more grooves can be formed into the sensor apparatus **401** to receive an edge of the sensor card **409** to securely position the sensor card **409** at a specified location or orientation if the sensor card **409** has a width greater than any axis of the sampling chamber **428** as shown in FIG. 4E.

The sampling chamber **428** can have a plurality of clear windows on the sidewalls to provide optical access to the sensor card **409.** Holes **429** formed into the body of the sensor apparatus **401** can be used to attach the sensor apparatus **401** to a console or system using screws or other fasteners as shown in FIG. 4D. The sampling chamber **428** extends longitudinally along a length of the receiving slot **402** of the sensor apparatus **401.** The receiving slot **402** can extend beyond the sampling chamber **428** and terminate in a fastening mechanism to securely hold the sensor card **409,** such as the indentation **430.** The sampling chamber **428** can mix fluids to improve fluid contact on the sensor card **409.** Notably, the sampling chamber **428** defines a volume such that a front side and a back side of the sensor card **409** can be exposed to fluid flow on both sides of the sensor card **409.** The sampled fluid can therefore simultaneously contact a first and second side (or front and back) of the sensor card **409** to advantageously increase the surface area on which fluid contacts sensor membranes or colorimetric materials in the sensor card **409.** The resulting mixing can result in improved sensing of the fluid by the sensor card **409.** The larger contact surface area of the sensor membranes or colorimetric materials result in a shorter response time for the sensor membranes or colorimetric materials to changes in the fluid composition.

As described, the sensor card **409** can have at least a pH sensor membrane and an ammonia sensor membrane. Further, the ammonia sensor membrane can be a low sensitivity or a high sensitivity membrane as described herein. The pH sensor and ammonia sensor membranes can change color based on a pH and/or ammonia concentration of a fluid flowing through the sampling chamber **428.** As described, the color change can be observed through the one or more clear windows positioned on the sidewall of the sampling chamber **428.** Temperature probe **422** can determine the temperature of the fluid within the sensor apparatus **401** for determination of total ammonia content based on the ammonia concentration and pH. Electrical connector **424** provides the electrical connection from the temperature probe **422** to the sensor apparatus **401.** The sensor apparatus is not limited to detection of pH and/or ammonia, and can detect any substance that can produce a detectable change in a substrate on a sensor card. Any colorimetric material can be included in the sensor card for detection of any substance.

In FIG. 4F, one non-limiting example of a light emitting source is shown as LED array **431** connected to the system by electrical connector **417.** The LED array **431** can transmit light through the sensor card **409** by shining a light onto a first side of the sensor card **409** seated inside the receiving slot **402.** The light can be directed through the one or more clear windows in the sidewall of the sampling chamber **428.** The light emitting source can be any source of light at any wavelength or color capable of shining light onto the sensor card **409.** In a preferred embodiment, the LED provides white light; however, any color or wavelength of light can be used. In a preferred embodiment, the light emitting source uniformly transmits light onto one side of the sensor card **409** such that a camera **406** (shown in FIG. 4A) positioned on an opposite side of the sensor apparatus **401** can detect changes on an opposite side of the sensor card **409** via one or more clear windows. In general, the clear windows for the LED array **431** and camera **406** are antipodal to each other. However, the LED array **431** can be positioned at any part of the apparatus capable of providing uniform light to the sensor card **409,** including direct and side-firing or side-emitting LEDs. The camera **406** can be any appropriate photodetector, spectrophotometer, or photosensor known to those of ordinary skill in the art. The camera **406** can transmit the image or sensed output to a processor for determining the pH or ammonia level of a fluid. The camera or photodetector **406** can also detect fluorescent light emitted from the sensor card. For detection of fluorescent light, an optical bandpass filter can be included in front of the camera to allow the emitted fluorescent light to pass to the camera while blocking any transmitted light from the LED array. The camera can detect any change in the light transmitted including the wavelength of light, the mean intensity of light, the variation in intensity of light, and the pixel location in an image produced by the camera. Variation in intensity of light and pixel location allow the automatic detection of the sensor membrane location in the image captured by the photodetector for image analysis, as well as detecting any holes in the sensor membranes, making image analysis easier due to the known variations in intensity and location. The camera can also be positioned within the sensor apparatus **401.** A waterproof camera can prevent damage to the camera from the fluid within the fluid sensor apparatus **401.**

In a preferred embodiment, the light is uniformly transmitted onto the sensor card **409.** Uniform lighting can result in even backlighting onto the sensor card **409.** Advantageously, uniform backlighting can improve accuracy of the sensed color changes on the sensor card **409.** The luminous intensity of the light on each sensor membrane can also be uniform, meaning that the power of the light emitted by the LED array in each direction to each sensor membrane is uniform. The luminous flux, or the quantity of energy of the light transmitted onto each sensor membrane, can also be uniform, as can the illuminance, or luminous flux per area of the sensor membranes. The clear windows can be positioned on the sidewalls to provide uniform light dispersion. Diffuser films and a light cavity can also be included to provide uniform lighting. Diffuser films are thin films that evenly distribute transmitted light. Non-limiting examples of diffuser films include Brightness Enhancement Film (BEF), Dual Brightness Enhancement Film (DBEF), and Universal Diffuser Film 50 (UDF 50), available from 3M™, a Minnesota corporation. A light cavity is an arrangement of mirrors or other reflectors, such as white surfaces, that can form standing waves from transmitted light. The lights on the LED array **431** can be arranged in any shape, including rectangular, circular, or other shape, to cast light onto the sensor card **409** in a desired dispersion. The sensor membranes can be positioned on the sensor card **409** to align with light cast by the LED array **431.** The power supply for the LED array **431** can provide a stable current and voltage to increase light uniformity. Although illustrated as opposing the camera, the LED array **431** can be positioned anywhere on the fluid sensor apparatus **401,** including on any side of the fluid sensor apparatus **401.** A light guide can be included to allow light from an LED array positioned on a side of the fluid sensor apparatus **401** to be transmitted through the sensor card and onto the camera. A light guide is an apparatus that can transmit light in a defined path by means of total or near total internal reflectance.

Alternatively, the backlight settings can be computer controlled to optimize the backlight for each sensor membrane. The light from the LED array can be set at a first intensity, optimized for a first sensor membrane. The LED can then be switched to a second intensity, optimized for a second sensor membrane. The camera can take an image of each sensor membrane at the optimized backlighting.

In FIG.'s 4A and 4F, the camera **406** and LED array **431** can be placed on opposing sides of the receiving slot **402** of FIG. 4D such that for each pair of sampling holes, a first hole of the pair of sampling holes faces the light emitting source, and a second hole of the pair of sampling holes face the camera **406,** thereby potentially reducing hot spots formed on the sensor card **409.** A grating light valve (not shown) having an adjustable diffraction grating can be included to control the amount of light diffracted onto the camera **406.** The diffraction grating can be a set of equally spaced, narrow, parallel grates. The grates can disperse the light at different wavelengths, so that light intensity can be measured as a function of a particular wavelength. One or more light diffusive layers can also be included to diffuse the light shining on the sensing material of the sensor card **409** prior to detection by the camera **406.** Desirably, the clear windows can be free from scratches that degrade sensor performance. In one non-limiting embodiment, to reduce scratches to the clear windows, the windows can be solvent polished. As shown in FIG. 4A, the camera **406** can transmit the image or other sensed output to a processor (not shown) in electronic communication with the camera **406** via electronic link **408.**

As described, the processor can determine the color of the pH sensor membrane and ammonia sensor membrane to determine the ph and/or ammonia concentration of the fluid flowing through the sensor apparatus **401.** The processor can detect any optically detectable change of any colorimetric material including in the sensor card. Electronics **407** of FIG. 4A can control the camera and the light emitting source. Although illustrated as having wired communication links between the camera, electronics, and processor, one of skill in the art will understand that any method of transmitting and receiving data can be used, including Bluetooth, Wi-Fi, or any other methods known in the art. The processor can receive data such as image data collected by the camera, and determine the intensity of pixels of a particular color in an image of the fluid sensor membranes. Experiments have shown green light to provide a good correlation between the fluid sensor membranes and the lab tested pH or ammonia concentration. The processor can determine the intensity of green pixels in the image produced by the camera. However, other colors such as red, blue, or any other suitable color can be used. The processor can then determine the ammonia concentration, the pH, and/or the total ammonia concentration in the fluid based on the intensity and color of the pixels detected. The processor can use lookup tables, algorithms or any other method for correlating the number of green pixels in the image produced by the camera to a pH or ammonia concentration. The processor can be housed within, or positioned outside of, the sensor apparatus **401.** The camera **406** can be operated under manual control or by a software application for controlling the exposure, gain and white balance.

As shown in FIG. 4A, fluid can enter the sensor apparatus **401** through a fluid inlet **403** and into the sampling chamber **428** of FIG. 4D. The fluid contacts the sensor card **409** seated in the receiving slot **402** of the sampling chamber **428.** The fluid can then exit the sampling chamber **428** through fluid outlets **404** and **405.** One of skill in the art will understand that one or more fluid inlets and outlets can be used. In a preferred embodiment, the two fluid outlets **404** and **405** advantageously improve fluid contact of the sensor membrane of the sensor card **409.** Notch **418** on fluid inlet **403,** notch **419** on outlet **404,** and notch **420** on outlet **405** can provide secured fastening of the fluid inlet **403** and fluid outlets **404** and **405** to tubing as needed.

The receiving slot **402** can include additional components to ensure that the detachable receiving slot cover **412** of FIG. 4C fits tightly over the receiving slot **402** and does not move as fluid is pumped into and through the sensor apparatus **401.** As illustrated in FIG.'s 4A and 4B, the receiving slot **402** can have an extended portion **410** that will contact the receiving slot cover **412** when closed. The extended portion **410** can include grooves **411** and **414** for receiving pins **413** and **415** when the receiving slot cover **412** is placed over the receiving slot **402.** The pins **413** and **415** engage with the grooves **411** to ensure the receiving slot cover **412** is properly placed and securely fastened on the sensor apparatus **401.**

To improve accurate measurements, the sensor card **409** can be fixed in position and/or orientation in the receiving slot **402.** Any suitable fastener to fix the receiving slot cover **412** to the sensor apparatus **401** is contemplated. Magnets can be placed within the receiving slot cover **412** and the sensor apparatus **401.** If the receiving slot cover **412** is closed, the magnets can provide a means to determine if cover **412** is closed over the receiving slot **402** on the sensor apparatus **401.** As shown in FIG. 4F, overhang **416** can provide support for the receiving slot cover **412** when closed. In FIG.'s 4D and 4E, opening **426** on extended portion **410** can provide for a fastener to be inserted through the receiving slot cover **412** to secure the receiving slot cover **412** onto the sensor apparatus **401.** The accuracy of the sensor can also be improved by making the interior of the receiving slot and/or sampling chamber non-reflective to prevent stray reflected light from interfering with the sensors. Similarly, any surface of the sensor card **409** can be non-reflective to improve accuracy and related light detection properties. The receiving slot, sampling chamber, and/or sensor card can be constructed from a non-reflective material, or colored in a non-reflective color, such as black.

In FIG. 4C, an annular bevel **427** can be formed on the receiving slot cover **412** to capture the sensor card and hold the sensor card securely locked in the sensor apparatus **401.** Screws **425** fasten the electronics and camera **406** to the sensor apparatus **401.** Alternative methods of securing components to the sensor apparatus **401** can be used, including adhesive, glue, bolts, or any other securing components known in the art. Holes **423** allow additional components and electronics to be added to the sensor apparatus **401.**

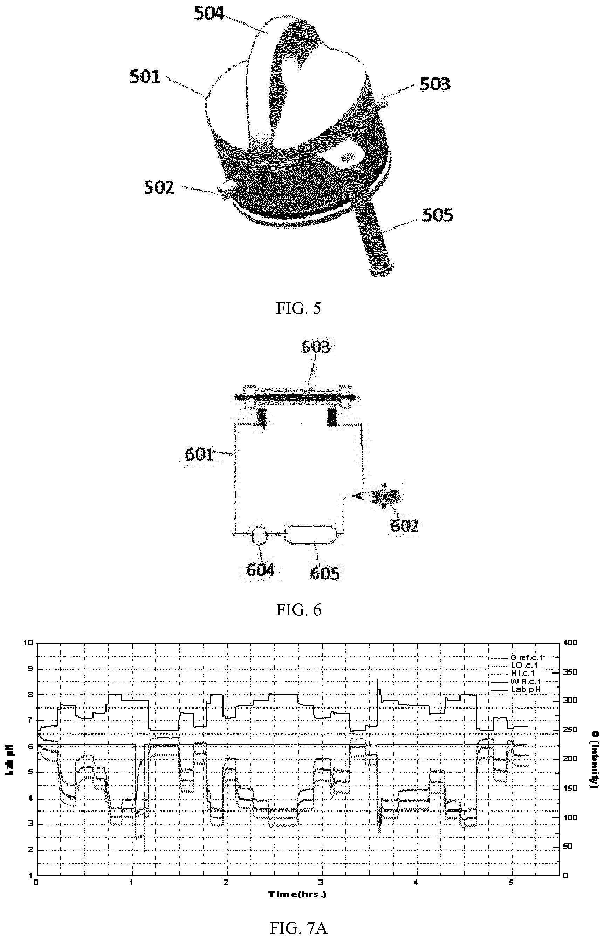

FIG. 5 illustrates a close-up view of a receiving slot cover **501** of the sensor apparatus. As described, the receiving slot cover **501** can include pins **502** and **503** to hold the receiving slot cover **501** in place on the sensor apparatus when engaged. The receiving slot cover **501** can also include a handle **504** to facilitate twisting of the receiving slot cover **501** for attachment and detachment of the receiving slot cover **501** to the sensor apparatus. A solenoid rod **505** can be included as a complimentary lock to a receiving hole in the sensor apparatus as a means to secure the receiving slot cover **501** when the receiving slot cover is open on the sensor apparatus and to prevent the cover from being removed during use. The solenoid rod **505** is insertable into the receiving hole of the sensor apparatus. Once inserted, the receiving slot cover **501** is fixed with respect to the sensor apparatus, ensuring that the sensor card does not move within the sensor apparatus, and that the receiving slot cover **501** does not move or disengage as fluid flows through the sensor apparatus. Alternatively, the solenoid rod **505** can be included in the sensor apparatus and insert into a receiving hole on the receiving slot cover **501.** Fixing the sensor card within the sensor apparatus provides the sensor and camera with a constant focal length, increasing the accuracy of the sensor. As described, a bevel (not shown) can be included on an interior surface of the receiving slot cover **501** for fixing the sensor card in place and to prevent insertion of the sensor card at a 180° rotation from the intended configuration. The bevel only allows the sensor card to be inserted into the sensor apparatus in a single configuration. A tapered edge of the sensor card is insertable into the bevel to lock the sensor card in a fixed position. The bevel can be sized and shaped to conform to a tapered edge of the sensor card, fixing the sensor card in position when placed into the bevel.

The removable sensor card can be a disposable sensor card for use with a non-disposable sensor apparatus. After each use, or if the sensor card is past useful life, the sensor card can be removed from the sensor apparatus and replaced with a new sensor card.

As described, the ammonia sensing region senses the amount of ammonia in a fluid by sensing the amount of gaseous ammonia (NH₃) contacting the ammonia sensor membranes. The total ammonia concentration of the fluid includes ammonia as well as ammonium ions (NH₄⁺), with ammonium ions accounting for the majority of the total ammonia. The pKₐ of ammonia depends on the temperature of the fluid and can be determined by a person skilled in the art for any temperature. With a known temperature, pH, and ammonia concentration, the ammonium ion concentration can be calculated using the Henderson-Hasselbalch equation. A temperature sensor can be included in the sampling chamber of pH and ammonia fluid sensor apparatus to allow calculation of total ammonia, or a separate temperature sensor can be included either upstream or downstream of the pH and ammonia fluid sensor apparatus in a fluid flow path. In general, the temperature sensor can refer to any device for measuring the temperature of a gas or liquid in a vessel, container, or fluid line. One of skill in the art will understand that a processor can determine the total ammonia concentration of the fluid based on the sensed ammonia concentration, the temperature, and the pH.

The sensor card and sensor apparatus can be used in any application where accurate measurement of ph and/or ammonia concentration is needed. The sensor card and sensor apparatus can measure the ph and/or ammonia concentration of a fluid either continuously or intermittently. The sensor apparatus can be fluidly connected to a fluid flow path, and images of the fluid sensor membranes can be taken by the camera as needed.

One non-limiting application of the sensor card is in dialysis. However, the sensor card can be used in any application with any clear aqueous liquid to be sensed. FIG. 6 illustrates a non-limiting embodiment of a dialysate flow path including the sensor apparatus **602** fluidly connected to the dialysate flow path. As dialysate is flowed through the sensor apparatus **602,** the fluid sensor membranes of the sensor card will be in fluid contact with the dialysate. One of skill in the art will understand that the dialysate flow path **601** illustrated in FIG. 6 is a simplified flow path, and that any number of additional components can be added as necessary. Dialysate pump **604** provides the driving force for pumping the dialysate through the dialysate flow path **601.** Dialysate in the dialysate flow path **601** passes through a dialyzer **603.** Blood from a patient is pumped through a blood flow path (not shown) and into the dialyzer **603.** Solutes in the blood and dialysate can cross a semipermeable membrane in the dialyzer **603** to move from a high concentration side of the membrane to a low concentration side of the membrane. A principal waste product removed during dialysis is urea, which moves from the patient's blood into the dialysate in the dialyzer **603.** The urea is removed from the dialysate in sorbent cartridge **605,** which can contain urease to catalyze the conversion of urea to ammonium ions and carbonate ions. The ammonium ions can be removed by a cation exchange membrane in the sorbent cartridge, as ammonia would be poisonous to pass back to the patient. Even though the ammonium ions are generally removed by the process, monitoring the presence of ammonium ions in dialysate fluid is desirable. One or more solute concentrations of a fluid can be determined by ammonia or ammonium ion concentration along with the pH of the fluid. A total ammonia content of a fluid can be determined by ammonia or ammonium ion concentration along with the pH of the fluid. The sensor apparatus **602,** containing a sensor card as described, can determine the ammonia level and ensure that the dialysate does not have an ammonia level in excess of a predetermined limit. The sensor apparatus **602** can be placed downstream of the sorbent cartridge **605** and upstream of the dialyzer **603,** allowing the ammonia level and pH of the dialysate to be determined after conversion of urea to ammonium ions, but prior to passing the dialysate back through the dialyzer **603.** The sensor apparatus **602** can be used to determine the ph and/or ammonia level in any fluid used in dialysis, including a dialysis fluid, a peritoneal dialysis fluid, a hemodialysis fluid, or a rinseback fluid. Although illustrated in FIG. 6 as a hemodialysis system, the sensor apparatus can also be used in peritoneal dialysis to determine the ph and/or ammonia level of any peritoneal dialysis fluid. The sensor apparatus **602,** containing a sensor card as described, can determine the ammonia concentration and ensure that the dialysate does not have an ammonia concentration in excess of a predetermined limit. The sensor apparatus **602** can be placed downstream of the sorbent cartridge and upstream of the dialyzer, allowing the ammonia concentration and pH of the dialysate to be determined after conversion of urea to ammonium ions, but prior to passing the dialysate back through the dialyzer **603.**

The sensor apparatus and sensor card can also be used to detect substances in gaseous fluids in addition to aqueous solutions. For example, when used to detect ammonia, ammonia gas in an environment can produce a detectable change in the ammonia sensing membranes in either the gaseous or solution state. As a non-limiting example, the fluid sensor apparatus can be used to detect ammonia in a refrigerated room where ammonia is used as the refrigerant. Air can flow over the sensors within the fluid sensor apparatus, and the presence of ammonia will produce a detectable change in the ammonia sensing membranes. The air flow through the fluid sensor apparatus can be active or passive. A fan can be included in the fluid sensor apparatus for active gas flow across the sensors.

To test the accuracy of the sensor apparatus, several experiments were conducted. For each experiment, two parallel sensor cells were tested in each run. The sensor cards used had three identical films (pH, NH₃ low sensitivity, or NH₄ high sensitivity), as well as a color reference sensing region. The test setup provided six replicated measurements on a single type of sensor film per run. The sensor cards were preconditioned to simulate the system start up. The preconditioning included agitating the sensor cards in 35 mM NaOH and 10% citric acid at room temperature for 12 minutes, agitating the sensor cards in 35 mM NaOH at room temperature for 9 minutes, agitating the sensor cards in 35 mM NaOH in phosphate buffered saline (PBS) at 37°C for 5 minutes, and agitating the sensor cards in PBS at 37°C for 15 minutes. The test runs were conducted in phosphate buffered saline (PBS) at 37°C, and a flow rate of 325 ml/min, unless otherwise stated. Previous tests have shown that sensor performance in PBS is the same as in simulated dialysate. The pH of the PBS was controlled by addition of HCl or NaOH. The ammonia concentration was controlled by addition of ammonium chloride. As such, the ammonium chloride concentration was only adjusted upward, however, the ammonia concentration can move up or down depending on the pH and temperature. The test runs were conducted for between 5-10 hours, depending on the number of data points collected. The pH was measured vs. a lab reference. The ammonia concentration is computed assuming the ammonium chloride concentration and the pH values. The assumptions correlated well with the true ammonium chloride concentration as determined by testing of collected samples. The images were collected every four seconds and the red, green, and blue values (RGB) determined for the regions of interest (mean ROI, 1500 pixels). Each test point was stabilized for 1 minute and collected for a minimum of three minutes. The RGB values are the average of the mean ROI values over the three minutes.

### Experiment 1

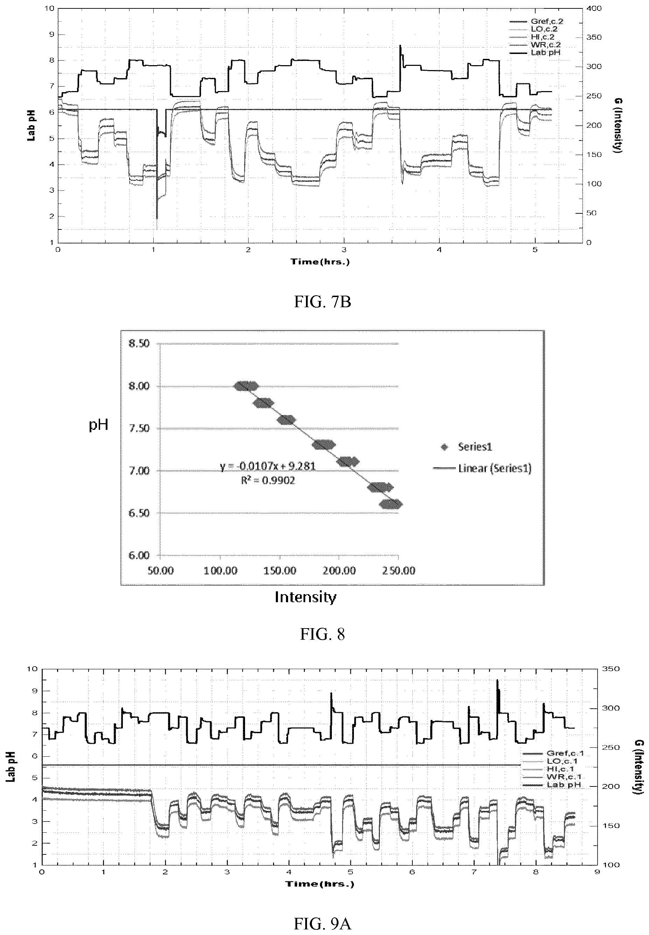

Prior test results established that detecting green light can provide a high degree of accuracy for the sensor cards. FIG.'s 7A and 7B illustrate the detected intensity of green light as a function of the pH of the fluid and time for two different sensor cards. The top black line in each graph is the lab tested pH of the fluid. The straight black line represents a green colored reference sensing region on the sensor cards. The light gray, medium gray, and dark gray lines at the bottom of the graphs are the detected green light intensity for each of the three pH sensor membranes. As illustrated in FIG.'s 7A and 7B, the intensity of the green light correlates well with the lab tested pH of the fluid for each sensor card. However, the green light as detected for each of the pH sensor membranes varied, as illustrated by the three different green lines in each graph. Further, a difference exists in the detected green light intensity between each sensor card, as illustrated in a comparison of FIG. 7A with FIG. 7B. The differences are likely due to non-uniform backlighting of the sensor films at each sensor film location and between fluid sensor apparati.

FIG. 8 illustrates the correlation between green light intensity as detected by the camera described and the lab tested pH values of the fluid for a pH sensor card. The green light as detected for each of the three pH sensor membranes on the sensor card is shown as the data points in FIG. 8. As illustrated in FIG. 8, the green light intensity increases with decreasing pH in a largely linear fashion. However, significant spread exists for each of the lots at each test point, indicating different intensities detected for each pH sensor membrane, likely due to non-uniform backlighting. A linear regression for the sensor card provided the change in green light intensity as a function of pH to be y = -0.0107x + 9.281 with an R² value of 0.9902.

### Experiment 2

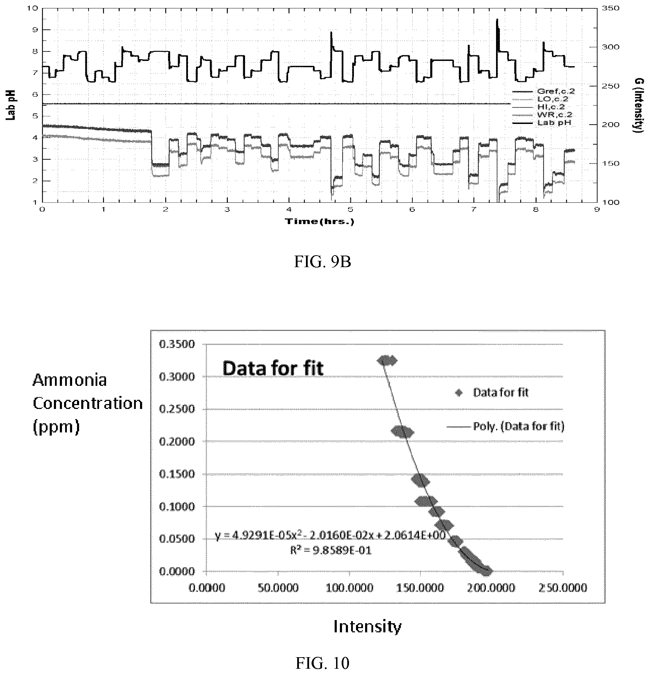

FIG.'s 9A and 9B illustrate the detected intensity of green light as a function of the pH of the fluid and time, as detected for a low sensitivity ammonia sensor membrane. The graph in FIG. 9A was obtained using a first sensor card with three ammonia sensing membranes, and the graph in FIG. 9B was obtained using a second sensor card with three ammonia sensing regions. The ammonia level in the fluid is altered as a function of the pH. The top black line in each graph is the lab tested pH of the fluid. The straight black line represents a green colored reference sensing region on the sensor cards. The light gray, medium gray, and dark gray lines on the bottom of the graph are the detected green light intensity for each of the three low sensitivity ammonia sensor membranes on each sensor card. As illustrated in FIG.'s 9A and 9B, the intensity of the green light correlates well with the lab tested pH of the fluid, and thus with the ammonia level. However, the green light as detected for each of the low sensitivity ammonia sensor membranes varied, as illustrated by the three different green lines in each of FIG.'s 9A and 9B. Further, a difference exists in the detected green light intensity between each sensor card, as illustrated in a comparison of FIG. 9A with FIG. 9B. These differences are due to non-uniform backlighting.

FIG. 10 illustrates the correlation between green light intensity as detected by the camera described and the calculated ammonia values of the fluid for the low sensitivity ammonia sensor membranes. The green light as detected for each of the three low sensitivity ammonia sensor membranes is shown as the data points in FIG. 10. As illustrated in FIG. 10, the green light intensity decreases with increasing ammonia levels. A small spread exists for each test point, indicating different intensities detected for each of the three low sensitivity ammonia sensor membranes on the sensor card, likely due to non-uniform backlighting. A polynomial regression provided the correlation between green light intensity and ammonia concentration as y = 4.9291^{∗}10⁻⁵(X²) - 2.0160^{∗}10⁻²(X) + 2.0614^{∗}10⁰, with an R2 value of 9.8589^{∗}10⁻¹. Based on the data presented in FIG. 10, the low sensitivity ammonia sensor membrane can be used for detecting the ammonia level at greater than 0.05 ppm.

### Experiment 3

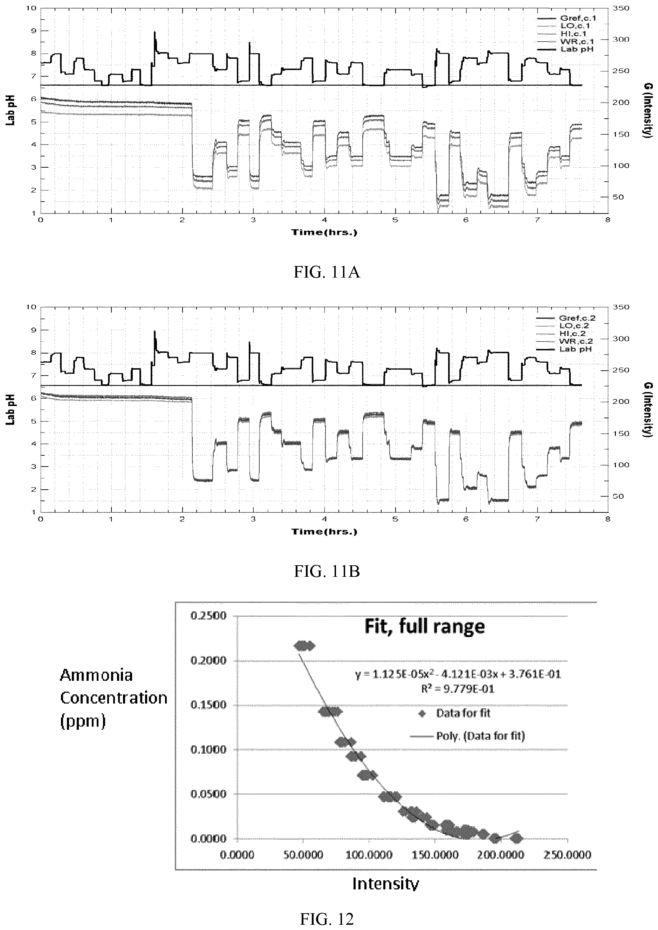

FIG.'s 11A and 11B illustrate the detected intensity of green light as a function of the pH of the fluid and time, as detected for a high sensitivity ammonia sensor membrane. The graph in FIG. 11A was obtained using a first sensor card with three high sensitivity ammonia sensing membranes, and the graph in FIG. 11B was obtained using a second sensor card with three high sensitivity ammonia sensing regions. The ammonia level in the fluid is altered as a function of the pH. The top black line in each graph is the lab tested pH of the fluid. The straight black line in each graph represents a green colored reference sensing region on the sensor cards. The light gray, medium gray, and dark gray lines at the bottom of each graph are the detected green light intensity for each of the three high sensitivity ammonia sensor membranes on each sensor card. As illustrated in FIG.'s 11A and 11B, the intensity of the green light correlates well with the lab tested pH of the fluid, and thus with the ammonia level for each of the two sensor cards. However, the green light as detected for each of the high sensitivity ammonia sensor membranes varied, as illustrated by the three different green lines in each graph, although the graph illustrated in FIG. 11B shows only a slight variation between high sensitivity ammonia sensor membranes. Further, a difference exists in the detected green light intensity between each sensor card, as illustrated in a comparison of FIG. 11A with FIG. 11B. The differences are likely due to non-uniform backlighting.

FIG. 12 illustrates the correlation between green light intensity as detected by the camera described and the calculated ammonia level of the fluid for a high sensitivity ammonia sensor card. The green light as detected for each of the three high sensitivity ammonia sensor membranes on the sensor card is shown as the data points in FIG. 12. As illustrated in FIG. 12, the green light intensity increases with decreasing ammonia level. However, significant spread exists at each test point, indicating different intensities detected for each pH sensor membrane, likely due to non-uniform backlighting, particularly at high ammonia levels. A polynomial regression for the sensor card illustrated in FIG. 12 provided the change in green light intensity as a function of ammonia concentration to be y = 1.125^{∗}10⁻⁵(X²) - 4.121^{∗}10⁻³(X) + 3.761^{∗}10⁻¹, with an R² value of 9.779^{∗}10⁻¹.

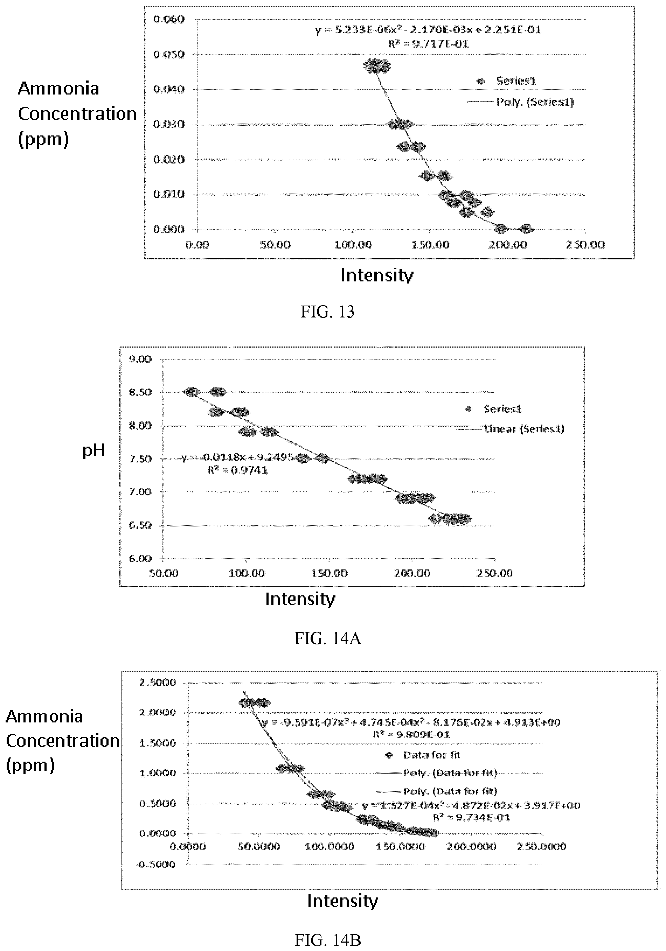

FIG. 13 illustrate the correlation between green light intensity as detected by the camera described and the calculated ammonia level of the fluid for a high sensitivity ammonia sensor card at ammonia levels less than 0.05 ppm. The green light as detected for each of the three high sensitivity ammonia sensor membranes is shown as the data points in FIG. 13. As illustrated in FIG. 13, the sensor cards are more accurate at ammonia levels less than 0.05 ppm. A polynomial regression for the sensor card, illustrated in FIG. 13 provided the change in green light intensity as a function of ammonia concentration to be y = 5.233^{∗}10⁻⁶(X²) - 2.170^{∗}10⁻³(X) + 2.251^{∗}10⁻¹, with an R² value of 9.717^{∗}10⁻¹.

### Experiment 4

The performance of the pH sensor membrane and low sensitivity ammonia sensor membrane over an extended range was also investigated. FIG. 14A illustrates the intensity of green light transmitted through the pH sensor membranes at a pH range from 6.5 to 8.0. The pH sensor card used in FIG. 14A included three pH sensor membranes, and the data from each pH sensor membrane is included in FIG. 20A. As illustrated in FIG. 14A, the pH sensor membrane is capable of sensing the pH of the fluid over the entire range tested, and should be capable within the entire range of the pH paper, or between 6.5 and 10. A linear regression for the sensor card illustrated in FIG. 14A provided the change in green light intensity as a function of pH to be y = -0.0118x + 9.2495 with an R² value of 0.9741 when fit for the entire pH range.

FIG. 14B illustrates the intensity of green light transmitted through the low sensitivity ammonia sensor membrane at a total ammonia level range of 0 to 20 ppm total ammonia, or 0 to 2.5 ppm ammonia. The ammonia sensor card used in FIG. 14A included three low sensitivity ammonia sensor membranes, and the data from each low sensitivity ammonia sensor membrane is included in FIG. 14B. As illustrated in FIG. 14B, the low sensitivity ammonia sensor membrane is capable of accurately measuring the ammonia level up to 20 ppm. At higher concentrations, the accuracy of the low sensitivity ammonia sensor membrane degrades and provides less signal change per ppm of ammonia. FIG. 14B includes two separate polynomial regressions, with a first polynomial regression for higher ammonia concentrations providing the change in green light intensity as a function of ammonia concentration to be y = - 9.591^{∗}10⁻⁷(X³) + 4.745^{∗}10⁻⁴(X²) - 8.176^{∗}10⁻²(X) + 4.913^{∗}10⁰, with an R² value of 9.809^{∗}10⁻¹. The second polynomial regression for lower ammonia concentrations provided the change in green light intensity as a function of ammonia concentration to be y = 1.527^{∗}10⁻⁴(X²) - 4.872^{∗}10⁻²(X) + 3.917^{∗}10⁰, with an R² value of 9.734^{∗}10⁻¹. The performance of both sensor membranes can be improved with optimized backlighting and sensor window positions.

### Experiment 5

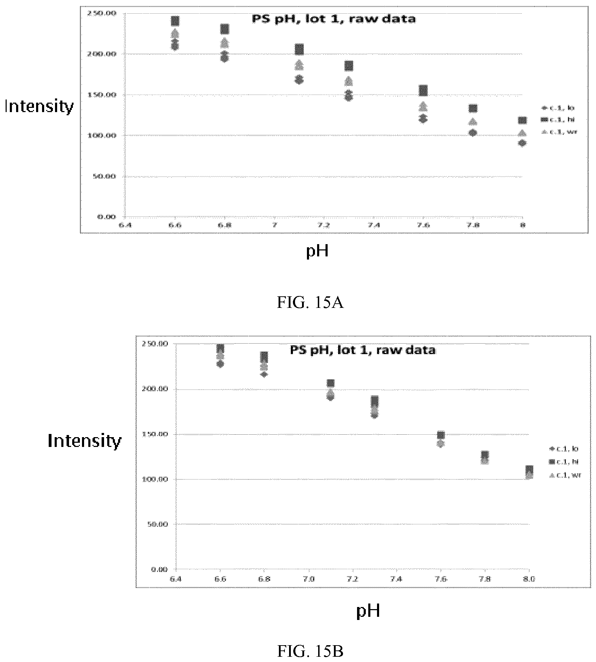

To test the effects of uniform backlighting on each of the pH sensor membranes in a sensor card having three pH sensor membranes, an LED array was constructed for the sensor apparatus that provides a uniform backlight on all three sensor membranes. FIG. 15A illustrates the intensity of green light detected from each of the three pH sensor membranes without uniform backlighting. A first pH sensor membrane at a first location on the sensor card is represented as the squares in FIG. 15A and labeled c.1 hi, a second pH sensor membrane at a second position on the sensor card is represented as the triangles and labeled c.1 wr, and a third pH sensor membrane at a third location on the sensor card is represented by the diamonds and labeled c.1 lo. FIG. 15B illustrates the intensity of green light detected from each of the three pH sensor membranes with uniform backlighting. A first pH sensor membrane at a first location on the sensor card is represented as the squares in FIG. 15B and labeled c.1 hi, a second pH sensor membrane at a second location on the sensor card is represented as the triangles and labeled c.1 wr, and a third pH sensor membrane at a third location on the sensor card is represented by the diamonds and labeled c.1 lo. As illustrated by a comparison of FIG. 15A to 15B, more uniform backlighting provides less variation between the three pH sensor membranes, as the intensities of light transmitted through each of the pH sensing membranes of FIG. 15B are closer together. However, the backlight used was not uniform enough to remove all variation.

### Experiment 6

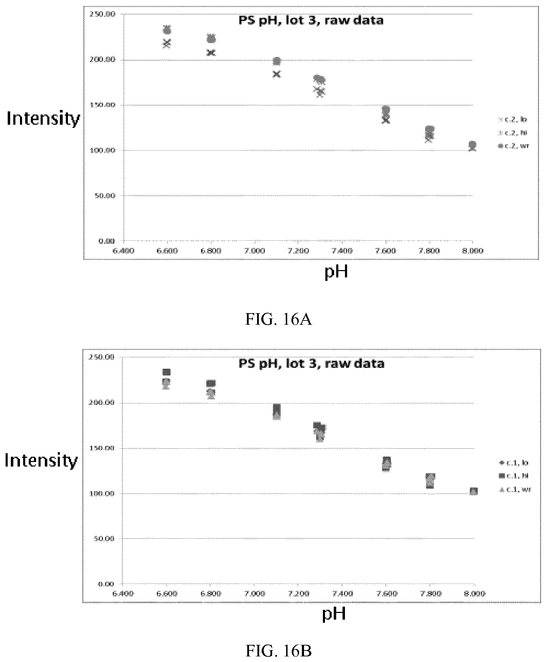

To test whether the remaining variation in the pH sensor membranes may be due to spherical lens aberration, the pH sensor membranes and windows on a sensor card were positioned symmetrically about the imaging axis of the camera lens, with each pH sensor membrane and window equidistant from the imaging axis. FIG. 16A illustrates the intensity of green light detected from each of the three pH sensor membranes without a uniform backlight, but with a symmetric pH sensor membrane and window arrangement. The light gray X's in FIG. 16A represent the detected light transmitted through a first pH sensor membrane at a first location on the sensor card and are labeled c.2 hi, the circles represent the light transmitted through a second pH sensor membrane at a second location on the sensor card and are labeled c.2 wr, and the dark gray X's represent the light transmitted through the third pH sensor membrane at a third position on the sensor card and are labeled c.2 lo. FIG. 16B illustrates the intensity of green light detected from each of the three pH sensor membranes with a uniform backlight and with a symmetrical placement of the pH sensor membranes about axis perpendicular to the sensor card, and equidistant to the axis of the sensor card. The diamonds in FIG. 16B represent the detected light transmitted through a first pH sensor membrane at a first position on the sensor card labeled c.1 lo, the squares represent the light transmitted through a second pH sensor membrane at a second position on the sensor card labeled c.1 hi, and the triangles represent the light transmitted through the third pH sensor membrane at a third position on the sensor card labeled c.1 wr. A symmetrical pH sensor membrane and window placement provides superior uniformity in the intensity of green light detected even without uniform backlighting, as the data points from each of the pH sensor membranes in FIG. 16A are closer together than without a symmetrical placement, as illustrated in a comparison of FIG. 15A and 16A. The combination of uniform backlighting and a symmetrical pH sensor membrane and window placement provides the most consistent light intensity across the three pH sensor membranes, as illustrated in a comparison of FIG.'s 16A and 16B, with the data in FIG. 16B providing the closest match between the three sensor membranes.

### Experiment 7

Experiments 1-6 illustrate sensors that detect the intensity of green light transmitted through each of the sensor membranes. FIG. 17 illustrates the correlation between red, blue, and green light and the pH. The data illustrated in FIG. 17 was taken at 37°C at 325 mL/min in PBS. The top graph of FIG. 17 is the correlation between red light transmitted through the sensor card and the pH for three different pH sensor membranes on a single sensor card, the middle graph is the correlation between green light transmitted through the sensor card and pH for three different pH sensor membranes on a single sensor card, and the bottom graph is the correlation between blue light transmitted through the sensor card and pH for three different pH sensor membranes on a single sensor card. In each graph, three separate pH sensor membranes were used. Each graph shows the same three pH sensor membranes. Each graph shows the red, green, or blue data for each of the three pH sensor membranes as a solid line vs. time compared to a dotted line for lab pH vs. time. All three color signals respond to changes in pH, with the intensity of transmitted light inversely proportional to the pH. Experiments have shown the same results for ammonia sensor films. Although red, green, or blue light can be used, in a preferred embodiment the system uses green light because green light provides the highest signal vs. pH or ammonia change slope and thus the best sensitivity.

One skilled in the art will understand that various combinations and/or modifications and variations can be made in the described systems and methods depending upon the specific needs for operation, limited only by the appended claims. Features illustrated or described as being part of an aspect of the invention may be used in the aspect of the invention, either alone or in combination.

## Claims

1. A system for determining solute concentration and/or pH of a fluid, the system comprising:
a sensor card (101, 201, 1801, 2401, 2501, 2508, 2516, 2601, 2608, 2616), comprising:
at least two fluid sensor membranes (301, 303) comprising a colorimetric material;
at least one reference element with a surface comprising a reference sensing region (1806) having a solid colour;
a front carrier (307) overlaying a front side of the at least two fluid sensor membranes and at least one reference sensing region;
at least two first sampling holes (308, 310) positioned on the front carrier aligned over the front side of the fluid sensor membranes, wherein the at least two first sampling holes are equidistant to an axis perpendicular to the sensor card; and
a first reference sampling hole positioned on the front carrier aligned over the front side of the at least one reference sensing region, wherein the first reference sampling hole is positioned at the axis perpendicular to the sensor card; and
a light emitting source (431) configured to transmit light through the at least two fluid sensor membranes and the at least one reference element of the sensor card,
wherein the at least one reference element with the reference sensing region having the solid colour is configured to transmit a light emitted from the light source.

2. The system of claim 1, the sensor card (101, 201, 1801, 2401, 2501, 2508, 2516, 2601, 2608, 2616) further comprising:
a back carrier (306) overlaying a back side of the at least two fluid sensor membranes (301, 303) and a back side of the reference sensing region (1806);
at least two second sampling holes (311, 313) positioned on the back carrier aligned over the back side of the fluid sensor membranes; the first and second sampling holes opposedly positioned on the sensor card;
a second reference sampling hole positioned on the back carrier aligned over the back side of the reference sensing region; the first and second reference sampling holes opposely positioned on the sensor card.

3. The system of any preceding claim, wherein the at least two fluid sensor membranes (301, 303) are selected from the group consisting of a pH sensor membrane, a low sensitivity ammonia sensor membrane, and a high sensitivity ammonia sensor membrane, wherein the pH sensor membrane detects pH in a range of 6.8 to 7.8, the high sensitivity ammonia sensor membrane detects ammonia in a range of 1 ppm to 2 ppm, and the low sensitivity ammonia sensor membrane detects ammonia in a range of 1 ppm to 20 ppm.

4. The system of claim 2, further comprising a camera or photodetector (406) detecting the transmitted light; preferably wherein the first sampling holes (308, 310) face the light emitting source (431), and the second sampling holes (311, 313) face the camera or photodetector (406); preferably wherein the axis perpendicular to the sensor card (101, 201, 1801, 2401, 2501, 2508, 2516, 2601, 2608, 2616) is substantially aligned to a perpendicular center axis of a lens of the camera or photodetector.

5. The system of claim 1, wherein the at least two first sampling holes (308, 310) are positioned symmetrically about the axis perpendicular to the sensor card (101, 201, 1801, 2401, 2501, 2508, 2516, 2601, 2608, 2616).

6. The system of any preceding claim, wherein the sampling holes (308, 310, 311, 313) have a shape selected from the group of rectangular, ovoid, circular, triangular, arced, and combinations thereof.

7. The system of claim 1, wherein the colorimetric material detects any one of alkalinity, aluminum, ammonium, calcium, carbonate, chloride, chlorine, chlorine dioxide, chromate, color, copper, cyanide, fluoride, formaldehyde, hydrazine, iron, magnesium, manganese, nickel, nitrate, nitrite, oxygen, ozone, pH, phosphate, residual hardness, silicate, sulfate, sulfide, sulfite, total hardness, urea, zinc, or combinations thereof.

8. The system of claim 2, the sensor card (101, 201, 1801, 2401, 2501, 2508, 2516, 2601, 2608, 2616) further comprising:
a first adhesive layer (304) interposed between the front carrier (307) and the at least two fluid sensor membranes (301, 303); and
a second adhesive layer (305) interposed between the back carrier (306) and the at least two fluid sensor membranes; preferably wherein the first adhesive and second adhesive layers have a hole cut-out aligned to the first and second sampling holes (308, 310, 311, 313).

9. The system of any preceding claim, the sensor card (101, 201, 1801, 2401, 2501, 2508, 2516, 2601, 2608, 2616) further comprising a pressure equalizing hole (205) positioned through the sensor card; preferably wherein the sensor card has a thickness of between 0.5 and 3.0 mm.

10. The system of claim 2, wherein the front and back carrier (306, 307) are polypropylene, polyvinyl chloride, dyed polytetrafluoroethylene, ethylene tetrafluoroethylene, polyvinylidene difluoride, fluorinated ethylene propylene, polyethylene, polyimide, polyetheretherketone, or combinations thereof.

11. The system of claim 2, wherein the sensor card (101, 201, 1801, 2401, 2501, 2508, 2516, 2601, 2608, 2616) has a bar code (2412) fixed on either a surface of the front carrier (307) or the back carrier (306).

12. A method, comprising the steps of:
flowing a fluid over opposite sides of the sensor card (101, 201, 1801, 2401, 2501, 2508, 2516, 2601, 2608, 2616) of the system accordingly to any preceding claim, wherein a characteristic of the fluid triggers an optically observable change in the at least two fluid sensor membranes (301, 303);
transmitting a light through one side of the at least two fluid sensor membranes; and
detecting the optically observable change on an opposite side of the at least two fluid sensor membranes.

13. The method of claim 12, further comprising the step of:
determining any one of a pH or ammonia concentration based on the optically observable change of the at least two fluid sensor membranes (301, 303).

14. The method of claim 11 or claim 12, further comprising the step of:
uniformly transmitting the light onto the one side of the at least two fluid sensor membranes (301, 303) using an LED array.

15. The method of any of claims 12-14, wherein the fluid is dialysate and the at least two fluid sensor membranes (301, 303) are in fluid contact with a dialysate flow path (601); preferably further comprising the step of flowing the dialysate through a sorbent cartridge (605) prior to flowing the dialysate over opposite sides of the at least two fluid sensor membranes.

## Patentansprüche

1. System zur Bestimmung der Konzentration gelöster Stoffe und/oder des pH-Werts eines Fluids, wobei das System umfasst:
eine Sensorkarte (101, 201, 1801, 2401, 2501, 2508, 2516, 2601, 2608, 2616), umfassend:
mindestens zwei Fluidsensormembranen (301, 303), umfassend ein kolorimetrisches Material;
mindestens ein Referenzelement mit einer Oberfläche, die einen Referenzerfassungsbereich (1806) mit einer Volltonfarbe umfasst;
einen vorderen Träger (307), der eine Vorderseite der mindestens zwei Fluidsensormembranen und mindestens einen Referenzerfassungsbereich überlagert;
mindestens zwei erste Probenahmelöcher (308, 310), die auf dem vorderen Träger positioniert sind und über der Vorderseite der Fluidsensormembranen ausgerichtet sind, wobei die mindestens zwei ersten Probenahmelöcher gleich weit von einer Achse senkrecht zur Sensorkarte entfernt sind; und
ein erstes Referenzprobenahmeloch, das auf dem vorderen Träger positioniert ist und über der Vorderseite des mindestens einen Referenzerfassungsbereichs ausgerichtet ist, wobei das erste Referenzprobenahmeloch an der Achse senkrecht zur Sensorkarte positioniert ist; und
eine Licht emittierende Quelle (431), die konfiguriert ist, um Licht durch die mindestens zwei Fluidsensormembranen und das mindestens eine Referenzelement der Sensorkarte zu übertragen, wobei das mindestens eine Referenzelement mit dem Referenzerfassungsbereich mit der Volltonfarbe konfiguriert ist, um ein von der Lichtquelle emittiertes Licht zu übertragen.

2. System nach Anspruch 1, wobei die Sensorkarte (101, 201, 1801, 2401, 2501, 2508, 2516, 2601, 2608, 2616) ferner umfasst:
einen hinteren Träger (306), der eine Rückseite der mindestens zwei Fluidsensormembranen (301, 303) und eine Rückseite des Referenzerfassungsbereichs (1806) überlagert;
mindestens zwei zweite Probenahmelöcher (311, 313), die auf dem hinteren Träger positioniert sind und über der Rückseite der Fluidsensormembranen ausgerichtet sind; wobei das erste und das zweite Probenahmeloch gegenüberliegend auf der Sensorkarte positioniert sind;
ein zweites Referenzprobenahmeloch, das auf dem hinteren Träger positioniert ist und über der Rückseite des Referenzerfassungsbereichs ausgerichtet ist; wobei sich das erste und das zweite Referenzprobenahmeloch gegenüberliegend auf der Sensorkarte befinden.

3. System nach einem der vorhergehenden Ansprüche, wobei die mindestens zwei Fluidsensormembranen (301, 303) ausgewählt sind aus der Gruppe, bestehend aus einer pH-Sensormembran, einer Ammoniaksensormembran mit niedriger Empfindlichkeit und einer Ammoniaksensormembran mit hoher Empfindlichkeit, wobei die pH-Sensormembran den pH-Wert in einem Bereich von 6,8 bis 7,8 erfasst, die Ammoniaksensormembran mit hoher Empfindlichkeit Ammoniak in einem Bereich von 1 ppm bis 2 ppm erfasst und die Ammoniaksensormembran mit niedriger Empfindlichkeit Ammoniak in einem Bereich von 1 ppm bis 20 ppm erfasst.

4. System nach Anspruch 2, ferner umfassend eine Kamera oder einen Fotodetektor (406), die/der das durchgelassene Licht erfasst; wobei vorzugsweise die ersten Probenahmelöcher (308, 310) der Licht emittierenden Quelle (431) zugewandt sind und die zweiten Probenahmelöcher (311, 313) der Kamera oder dem Fotodetektor (406) zugewandt sind; wobei vorzugsweise die Achse senkrecht zur Sensorkarte (101, 201, 1801, 2401, 2501, 2508, 2516, 2601, 2608, 2616) im Wesentlichen zu einer senkrechten Mittelachse einer Linse der Kamera oder des Fotodetektors ausgerichtet ist.

5. System nach Anspruch 1, wobei die mindestens zwei ersten Probenahmelöcher (308, 310) symmetrisch um die Achse senkrecht zur Sensorkarte (101, 201, 1801, 2401, 2501, 2508, 2516, 2601, 2608, 2616) angeordnet sind.

6. System nach einem der vorhergehenden Ansprüche, wobei die Probenahmelöcher (308, 310, 311, 313) eine Form aufweisen, die aus der Gruppe von rechteckigen, eiförmigen, kreisförmigen, dreieckigen, bogenförmigen und Kombinationen davon ausgewählt ist.

7. System nach Anspruch 1, wobei das kolorimetrische Material Alkalität, Aluminium, Ammonium, Calcium, Carbonat, Chlorid, Chlor, Chlordioxid, Chromat, Farbe, Kupfer, Cyanid, Fluorid, Formaldehyd, Hydrazin, Eisen, Magnesium, Mangan, Nickel, Nitrat, Nitrit, Sauerstoff, Ozon, pH-Wert, Phosphat, Resthärte, Silikat, Sulfat, Sulfid, Sulfit, Gesamthärte, Harnstoff, Zink oder Kombinationen davon erfasst.

8. System nach Anspruch 2, wobei die Sensorkarte (101, 201, 1801, 2401, 2501, 2508, 2516, 2601, 2608, 2616) ferner umfasst:
eine erste Klebeschicht (304), die zwischen dem vorderen Träger (307) und den mindestens zwei Fluidsensormembranen (301, 303) angeordnet ist; und
eine zweite Klebeschicht (305), die zwischen dem hinteren Träger (306) und den mindestens zwei Fluidsensormembranen angeordnet ist; wobei vorzugsweise die erste Klebstoff- und die zweite Klebstoffschicht einen Lochausschnitt aufweisen, der auf das erste und das zweite Probenahmeloch (308, 310, 311, 313) ausgerichtet ist.

9. System nach einem vorhergehenden Ansprüche, wobei die Sensorkarte (101, 201, 1801, 2401, 2501, 2508, 2516, 2601, 2608, 2616) ferner ein Druckausgleichsloch (205) umfasst, das durch die Sensorkarte positioniert ist; wobei vorzugsweise die Sensorkarte eine Dicke zwischen 0,5 und 3,0 mm aufweist.

10. System nach Anspruch 2, wobei der vordere und hintere Träger (306, 307) Polypropylen, Polyvinylchlorid, gefärbtes Polytetrafluorethylen, Ethylentetrafluorethylen, Polyvinylidendifluorid, fluoriertes Ethylenpropylen, Polyethylen, Polyimid, Polyetheretherketon oder Kombinationen davon sind.

11. System nach Anspruch 2, wobei die Sensorkarte (101, 201, 1801, 2401, 2501, 2508, 2516, 2601, 2608, 2616) einen Strichcode (2412) aufweist, der an einer der Oberflächen des vorderen Trägers (307) oder des hinteren Trägers (306) befestigt ist.

12. Verfahren, umfassend die folgenden Schritte:
Fließen eines Fluids über gegenüberliegende Seiten der Sensorkarte (101, 201, 1801, 2401, 2501, 2508, 2516, 2601, 2608, 2616) des Systems gemäß einem vorhergehenden Anspruch, wobei eine Eigenschaft des Fluids eine optisch beobachtbare Änderung der mindestens zwei Fluidsensormembranen (301, 303) auslöst;
Durchlassen eines Lichts durch eine Seite der mindestens zwei Fluidsensormembranen; und
Erfassen der optisch beobachtbaren Änderung auf einer gegenüberliegenden Seite der mindestens zwei Fluidsensormembranen.

13. Verfahren nach Anspruch 12, ferner umfassend den folgenden Schritt:
Bestimmen eines pH-Werts oder einer Ammoniakkonzentration basierend auf der optisch beobachtbaren Änderung der mindestens zwei Fluidsensormembranen (301, 303).

14. Verfahren nach Anspruch 11 oder 12, ferner umfassend den folgenden Schritt:
gleichmäßiges Übertragen des Lichts auf die eine Seite der mindestens zwei Fluidsensormembranen (301, 303) unter Verwendung eines LED-Arrays.

15. Verfahren nach einem der Ansprüche 12 bis 14, wobei das Fluid Dialysat ist und die mindestens zwei Fluidsensormembranen (301, 303) in Fluidkontakt mit einem Dialysatströmungsweg (601) stehen; ferner vorzugsweise umfassend den Schritt des Fließens des Dialysats durch eine Sorptionsmittelkartusche (605) vor dem Fließen des Dialysats über gegenüberliegende Seiten der mindestens zwei Fluidsensormembranen.

## Revendications

1. Système de détermination de la concentration en soluté et/ou du pH d'un fluide, ledit système comprenant :
une carte de détecteur (101, 201, 1801, 2401, 2501, 2508, 2516, 2601, 2608, 2616) comprenant :
au moins deux membranes de détecteur de fluide (301, 303) comprenant un matériau colorimétrique ;
au moins un élément de référence comportant une surface comprenant une zone de détection de référence (1806) de couleur unie ;
un support avant (307) recouvrant une face avant des au moins deux membranes de détecteur de fluide et au moins une zone de détection de référence ;
au moins deux premiers trous d'échantillonnage (308, 310) positionnés sur le support avant aligné sur la face avant des membranes de détecteur de fluide, dans lequel les au moins deux premiers trous d'échantillonnage sont équidistants à un axe perpendiculaire à la carte de détecteur ; et
un premier trou d'échantillonnage de référence positionné sur le support avant aligné sur la face avant de l'au moins une zone de détection de référence, dans lequel le premier trou d'échantillonnage de référence est positionné sur l'axe perpendiculaire à la carte de détecteur ; et
une source électroluminescente (431) conçue pour transmettre de la lumière à travers les au moins deux membranes de détecteur de fluide et l'au moins un élément de référence de la carte de détecteur, dans lequel l'au moins un élément de référence comportant la zone de détection de référence de couleur unie est conçu pour transmettre une lumière émise par la source lumineuse.

2. Système selon la revendication 1, la carte de détecteur (101, 201, 1801, 2401, 2501, 2508, 2516, 2601, 2608, 2616) comprenant en outre :
un support arrière (306) recouvrant une face arrière des au moins deux membranes de détecteur de fluide (301, 303) et une face arrière de la zone de détection de référence (1806) ;
au moins deux seconds trous d'échantillonnage (311, 313) positionnés sur le support arrière aligné sur la face arrière des membranes de détecteur de fluide ; les premiers et seconds trous d'échantillonnage étant positionnés de façon opposée sur la carte de détecteur ;
un second trou d'échantillonnage de référence positionné sur le support arrière aligné sur la face arrière de la zone de détection de référence ; les premier et second trous d'échantillonnage de référence étant positionnés de façon opposée sur la carte de détecteur.

3. Système selon l'une quelconque des revendications précédentes, dans lequel les au moins deux membranes de détecteur de fluide (301, 303) sont choisies dans le groupe constitué par une membrane de détecteur de pH, une membrane de détecteur d'ammoniac à faible sensibilité et une membrane de détecteur d'ammoniac à haute sensibilité, dans lequel la membrane de détecteur de pH détecte le pH dans une plage comprise entre 6,8 et 7,8, la membrane de détecteur d'ammoniac à haute sensibilité détecte l'ammoniac dans une plage comprise entre 1 ppm et 2 ppm et la membrane de détecteur d'ammoniac à faible sensibilité détecte l'ammoniac dans une plage comprise entre 1 ppm et 20 ppm.

4. Système selon la revendication 2, comprenant en outre une caméra ou un photodétecteur (406) détectant la lumière transmise ; de préférence dans lequel les premiers trous d'échantillonnage (308, 310) font face à la source électroluminescente (431), et les seconds trous d'échantillonnage (311, 313) font face à la caméra ou au photodétecteur (406) ; de préférence dans lequel l'axe perpendiculaire à la carte de détecteur (101, 201, 1801, 2401, 2501, 2508, 2516, 2601, 2608, 2616) est sensiblement aligné sur un axe central perpendiculaire d'une lentille de la caméra ou du photodétecteur.

5. Système selon la revendication 1, dans lequel les au moins deux premiers trous d'échantillonnage (308, 310) sont positionnés symétriquement autour de l'axe perpendiculaire à la carte de détecteur (101, 201, 1801, 2401, 2501, 2508, 2516, 2601, 2608, 2616).

6. Système selon l'une quelconque des revendications précédentes, dans lequel les trous d'échantillonnage (308, 310, 311, 313) présentent une forme choisie dans le groupe de formes rectangulaire, ovoïde, circulaire, triangulaire, arquée, et leurs combinaisons.

7. Système selon la revendication 1, dans lequel le matériau colorimétrique détecte l'alcalinité et/ou l'aluminium et/ou l'ammonium et/ou le calcium et/ou le carbonate et/ou le chlorure et/ou le chlore et/ou le dioxyde de chlore et/ou le chromate et/ou la couleur et/ou le cuivre et/ou le cyanure et/ou le fluorure et/ou le formaldéhyde et/ou l'hydrazine et/ou le fer et/ou le magnésium et/ou le manganèse et/ou le nickel et/ou le nitrate et/ou le nitrite et/ou l'oxygène et/ou l'ozone et/ou le pH et/ou le phosphate et/ou la dureté résiduelle et/ou le silicate et/ou le sulfate et/ou le sulfure et/ou le sulfite et/ou la dureté totale et/ou l'urée et/ou le zinc et/ou leurs combinaisons.

8. Système selon la revendication 2, la carte de détecteur (101, 201, 1801, 2401, 2501, 2508, 2516, 2601, 2608, 2616) comprenant en outre :
une première couche adhésive (304) interposée entre le support avant (307) et les au moins deux membranes de détecteur de fluide (301, 303) ; et
une seconde couche adhésive (305) interposée entre le support arrière (306) et les au moins deux membranes de détecteur de fluide ; de préférence dans lequel la première couche adhésive et la seconde couche adhésive présentent une découpe de trou alignée sur les premiers et seconds trous d'échantillonnage (308, 310, 311, 313).

9. Système selon l'une quelconque des revendications précédentes, la carte de détecteur (101, 201, 1801, 2401, 2501, 2508, 2516, 2601, 2608, 2616) comprenant en outre un trou d'égalisation de pression (205) positionné à travers la carte de détecteur ; de préférence dans lequel la carte de détecteur présente une épaisseur comprise entre 0,5 et 3,0 mm.

10. Système selon la revendication 2, dans lequel les supports avant et arrière (306, 307) sont du polypropylène, du chlorure de polyvinyle, du polytétrafluoroéthylène teint, du tétrafluoroéthylène d'éthylène, du difluorure de polyvinylidène, de l'éthylène-propylène fluoré, du polyéthylène, du polyimide, du polyétheréthercétone, ou leurs combinaisons.

11. Système selon la revendication 2, dans lequel la carte de détecteur (101, 201, 1801, 2401, 2501, 2508, 2516, 2601, 2608, 2616) comporte un code à barres (2412) fixé sur une surface du support avant (307) ou du support arrière (306).

12. Procédé comprenant les étapes suivantes :
l'écoulement d'un fluide sur les faces opposées de la carte de détecteur (101, 201, 1801, 2401, 2501, 2508, 2516, 2601, 2608, 2616) du système selon l'une quelconque des revendications précédentes, dans lequel une caractéristique du fluide déclenche un changement optiquement observable des au moins deux membranes de détecteur de fluide (301, 303) ;
la transmission d'une lumière à travers une face des au moins deux membranes de détecteur de fluide ; et
la détection du changement optiquement observable sur une face opposée des au moins deux membranes de détecteur de fluide.

13. Procédé selon la revendication 12, comprenant en outre l'étape suivante :
la détermination d'un pH ou d'une concentration en ammoniac sur la base du changement optiquement observable des au moins deux membranes de détecteur de fluide (301, 303).

14. Procédé selon la revendication 11 ou 12, comprenant en outre l'étape suivante :
la transmission uniforme de la lumière sur l'autre face des au moins deux membranes de détecteur de fluide (301, 303) à l'aide d'un réseau de LED.

15. Procédé selon l'une quelconque des revendications 12 à 14, dans lequel le fluide est un dialysat et les au moins deux membranes de détecteur de fluide (301, 303) sont en contact fluidique avec un trajet d'écoulement de dialysat (601) ; de préférence comprenant en outre l'étape d'écoulement du dialysat à travers une cartouche de sorbant (605) avant l'écoulement du dialysat sur les faces opposées des au moins deux membranes de détecteur de fluide.
